# EUROPEAN PATENT APPLICATION

(11) **EP 2 027 871 A2**
(43) Date of publication of application: **25.02.2009**
(21) Application number: 07714492.1
(22) Date of filing: 19.02.2007
(51) Int. Cl.: A61K 38/43, A61K 45/00, A61P 35/00

(54) **NOVEL ANTICANCER CATHEPSIN PREPARATION AND ANTICANCER AGENT FOR COMBINATION THERAPY AGAINST CANCER USING THE SAME**

(30) Priority: 17.02.2006 JP 2006040447
(71) Applicant: Kyushu University, National University Corporation, Higashi-ku Fukuoka-shi Fukuoka 812-8581 (JP)
(72) Inventor: YAMAMOTO, Kenji, Fukuoka-shi, Fukuoka 8128581 (JP); KAWAKUBO, Tomoyo, Fukuoka-shi, Fukuoka 8128581 (JP)
(74) Representative: Armstrong, Iain Cheshire
(86) International application number: PCT/JP2007/052966
(87) International publication number: WO 2007/094495

(57) **Abstract**

The anticancer cathepsin formulation of the present invention contains cathepsin E and/or an active fragment comprising or containing an active site thereof as an effective ingredient.

The anticancer cathepsin formulation of the present invention can suppress the growth of cancer cells alone or prevent metastasis thereof without adversely affecting normal cells and induce apoptosis of cancer cells. As an alternative, the anticancer cathepsin formulation of then present invention can be combined with an anticancer agent and enhance sensitivity of the anticancer agent that itself does not demonstrate sensitivity to the anticancer agent and improving a therapy effect to a remarkable extent by the enhanced sensitivity action of the anticancer agent. Further, the enhancement of sensitivity of the anticancer agent permits a decrease in a dose of the anticancer agent resulting in a remarkable decrease of side effects, etc. of the anticancer agent.

## Description

### TECHNICAL FIELD

The present invention relates to a novel anticancer cathepsin formulation and an anticancer agent for use in combined anticancer therapy. More particularly, the present invention relates to an anticancer cathepsin formulation with cathepsin E and/or an active fragment comprising or containing an active site thereof as an active ingredient, and a novel anticancer cathepsin formulation containing an anticancer agent as an active ingredient in combination therewith, a novel anticancer cathepsin formulation capable of enhancing sensitivity of an anticancer agent to cancer by combined treatment with the anticancer agent, as well as a method for enhancing sensitivity of the anticancer agent and a method for cancer therapy using the anticancer agent.

### BACKGROUND TECHNOLOGY

Hitherto, a large number of anticancer agents for the treatment of cancers have been developed. These anticancer agents, however, have great drawbacks that they make an attack on cancer cells as well as normal cells, leading to damaging and killing both of them. Although until now there have been studied a number of anticancer substances that demonstrate cancer cells solely without an attack on normal cells, some substances that have shown activities at a laboratory level have been found, however, nothing that can exhibit activities to cancer cells but not to normal cells has yet been commercially available.

Many attempts have also been made of a combination therapy using several kinds of anticancer agents because many anticancer agents cannot demonstrate an adequate level of anticancer activities when administered solely. In many cases, however, such attempts do not provide sufficient effects under present conditions.
In addition to the above combination therapy using plural anticancer agents having different action mechanisms, a combination therapy of an anticancer agent with an agent having no anticancer action in itself has also been proposed. This combination therapy does not demonstrate sufficiently high effect on cancer.
Furthermore, a combination therapy of anticancer agents with various compounds is proposed (see Non-Patent Literature #1, #2 and #3), however, it can be said that such a therapy does not show adequate effects on cancer in many cases.

As the effects on cancer treatment could be improved in either combination therapy if sensitivity of the anticancer agents could be enhanced, various improvements of combination therapy have been demanded.

In the present description, terms "cancer" and "tumor" as well as related terms are used in less strict meaning and in exchangeable ways. It is to be understood, accordingly, that, for instance, when the word "cancer" is used solely, it is also used to encompass the meaning of "tumor" unless otherwise defined or except it is interpreted in different meanings from the context. It is likely understood that, when the word "tumor" is used solely, it is also used to encompass the meaning "cancer" unless otherwise defined or except it is so interpreted from the context.

Cathepsins belong to a group of acid proteinases, which are distributed in a wide range from higher animals to microorganisms and bear significant biological functions including intracellular and extracellular protein metabolisms, processing, and so on. Cathepsin D and cathepsin E arc present as aspartic proteinase family members in the endosome/lysosome system of higher animals. As aspartic proteinases are involved in the metabolism and processing of the intracellular and extracellular proteins, a variation with their activity levels is considered to lead to a variety of disease conditions including abnormality of blood pressure, gastric ulcer, cancer, and so on.

Among these cathepsins, cathepsin E is an intracellular aspartic proteinase distributing in a limited fashion in the epithelium of digestive organs such as stomach and intestinal tract, lymphoid tissues, urinary tissues, blood cells and skin. Cathepsin E is contained in the highest amount in the stomach among all the tissues than cathepsin D. The intracellular location of cathepsin E is apparently different from that of cathepsin D, and is tissue- and cell-specific. The membranous localization of cathepsin E is observed in erythrocytes, osteoclasts, proximal convoluted tubules, etc. The endolysosomal localization of cathepsin E was observed in antigen presenting cells such as microglia and macrophage. Cathepsin E is also found in the endoplasmic reticulum and Golgi complex in many other peripheral tissues. Studies so far made reveal that cathepsin E exerts the proteinase activity in the form of a mature structure only when it is located in the endosome/lysosome system in almost all cells except erythrocytes. Cathepsin E is remarkably increased in the immune cells such as microglia and macrophage at mRNA and protein levels in response to certain stimuli added to the cells, such as interferon-γ, lipopolysaccharide or the like, and a significant amount of the enzyme is secreted outside these cells as an active enzyme. Therefore, a great attention is focused on a close connection between these cell functions and the extracellular cathepsin E.

It can be noted that cathepsin E is apparently present in the neurons of old rats in which lipofuscin and C-terminal fragments of APP (amyloid precursor protein) are accumulated, although it can little be detected in the neurons of young rats. In the rat brain with the forebrain ischemia or administered with kainic acid therein, a remarkable increase of the expression of cathepsin E is recognized in the degenerating neurons present in the vulnerable brain regions against these stresses and in activated microglia accumulated in response to these stresses. These results imply a significant role cathepsin E during the course of execution of death of the neurons.

Further, it is known that cathepsin E is expressed predominantly in the immune cells and it is the proteinase secreted by the activated macrophage. The physiological function of this protein in the living body is left non-clarified yet in many respects. Studies so far conducted by the present inventors made it apparent that cathepsin E cuts off and releases tumor necrosis factor (TNF)-related apoptosis inducing ligand (TRAIL) in the form of a soluble molecule from surfaces of cancer cells, thereby inducing apoptosis specifically to various cancer cells and resulting in the cancer growth arrest and in the suppression of cancer metastasis and, in addition, that it causes no such events whatsoever in normal cells. It is found, accordingly, that cathepsin E has a novel anticancer activity that cannot be recognized at all in the other cathepsin groups.

On the other hand, it can be noted that apoptosis plays a significant role in the exclusion of transformed cells and virus-infected cells outside the living body by way of immune response and it is a major decisive factor of the regression of tumor cells. Although the mechanism of apoptosis of tumor cells is not yet elucidated well, the control of propagation of tumor cells and the induction of apoptosis are found extremely significant in predicting a good prognosis for patients with cancers. Evidence so far accumulated implies that apoptosis of tumor cells is controlled, for example, by molecules of a TNF family including TNF-α, FasL (also called CD95L or Apo1L) and TRAIL (or called Apo2L) (Reference #1). These cytokines are all type II transmembrane proteins, and can induce cell signaling of apoptosis to the target cells by connecting to death receptors intrinsic on the cell surface. These members of the TNF family form a trimer for inducing apoptosis and binds to the trimer of the intrinsic receptor formed on the surface of the target cell thereby inducing apoptosis. However, as only TRAIL among the family members can induce apoptosis solely in various cancer cells without exerting any influence upon normal cells, the TRAIL attracts a strong interest in utility as an anticancer agent (References #2 and #3). Moreover, recent research indicates that various effecter cells infiltrating into tumor cells, such as granulocytes, macrophages, natural killer cells, B lymphocytes, T lymphocytes and so on, bear an essential role as an anti-tumor immune response to the tumor cells. Among these cells, the macrophage is considered to play the most significant role in a host defense function against cancers by its several functions including, for example, the suppression of cancer cells, the processing of antigens and the presentation to T4 lymphocytes as well as an increase of secretion of various cytokines such as TNF-α, IL-1, TL-8 and so on, which have a significant role in a non-speeifie host defense (References #4, #5 and #6). Therefore, the anticancer effect upon tumors are considered to be induced by at least two mechanisms, that is, the mechanism for inducing apoptosis specific to cancer cells through TRAIL and the mechanism for causing injuries in cancer cells by tumor-infiltrating macrophages.

Many studies so far made reveal that many proteinases involved in cancers, including, for example, lysosomal cathepsin B, L and D, metalloproteinases such as MMP-1 and MMP-9, etc., have accelerating effects on the multiplication, growth, intrusion or metastasis of tumors (References #7 and #8), and that the propagation of tumors is controlled by suppressing the activities of these proteinase (Reference #7), and so on. Therefore, the proteinases are considered to be an "evil" for the living body in respect of cancers. It is little known, however, of a "good" proteinase that has a function of suppressing propagation, growth and metastasis of cancers.

As have been previously described, cathepsin E is predominantly expressed in cells of the immune system, such as antigen-presenting cells, lymphocytes and so on (References #9, #10, #11) and increases its amount of expression due to a stress by activation by IFN-γ, LPS, etc., in macrophage, leading to the secretion of an active molecule in a large amount outside the cells (Reference #12). Unlike the other cathepsin enzymes, cathepsin E has also unique properties such as, for example, a mode of cell-specific location and a processing mechanism (Reference #13). The connection of this observation with the biological function of proteins, however, is not understood well. Recently, it has been reported that cathepsin E plays an important role in the processing of an exogenous antigen in the extrinsic antigen-presenting mechanism by MHC class II molecule (References #10, #14, #15, #16). It has further been made apparent that cathepsin E knockout (CatE^{-/-}) mice spontaneously develop atopic dermabtis-like skin lesions when reared under normal conventional conditions, although no changes are caused to happen under specific pathogen-free (SPF) conditions (Reference #17). CatE^{-/-} mice also increases sensitivity to bacterial infection (Reference #18). Furthermore, recently, the present inventors have made it apparent that the deficiency of cathepsin E induces accumulation of major lysosome membrane glycoproteins (e.g., LAMP-1 and LAMP-2) in the lysosome, thereby elevating a lysosomal pH in the macrophage and causing damages in the cell structure and functions (Reference #18). Based on these observations, the present inventors have made it apparent that cathepsin E is deeply involved in the immune response and greatly contributes to the maintenance of integrity of the living body. It is implied from recent results of analysis of the processing of the expression of genes and proteins involved in various cancers that cathepsin E may be useful as a biomarker for cancers, although the role of the cathepsin E in the carcinogenetic mechanism is not yet clear from prior art literature.

It has been reported that these cathcpsins could be used as a tumor marker for squamous cell carcinoma and so on (Patent Publication #4), however, there have been no reports of the observation and it is not known that cathepsin E itself has an anticancer activity and it can accelerate sensitivity to an anticancer agent.
[Patent Publication #1] WO 01/008698
[Patent Publication #2] Japanese Patent Publication 2003-530297
[Patent Publication #3] Japanese Patent Publication 2004-26811
[Patent Publication #2] Japanese Patent Publication 2005-117993

### DISCLOSURE OF INVENTION

### [Problems to be solved by Invention]

As a result of research on the specific action of cathepsin E, the present inventors have found that cathepsin E itself demonstrates an anticancer activity and enhances sensitivity to anticancer agents, in particular when combined with other anticancer agents. Therefore, the present invention has been completed on the basis of this finding.

The present invention as a major embodiment has the object to provide an anticancer cathepsin formulation containing cathepsin E and/or an active fragment comprising or containing its active site as an effective ingredient.

As another embodiment, the present invention has the object to provide an anticancer cathepsin formulation further containing an anticancer agent, which is capable of enhancing the action and effect of the anticancer agent, in combination with the above effective ingredient.

The present invention also has the object to provide a method for enhancing sensitivity of an anticancer agent by using the anticancer cathepsin formulation containing the cathepsin E and/or the active fragment comprising or containing its active site as well as the anticancer agent.

The present invention further has the object to provide a method for cancer therapy by treating the cancers with the above anticancer cathepsin formulation.

Moreover, the present invention has the object to provide a tumor necrosis factor (TNF) homotrimer formed with a member or members of a TNF family. [Means to solve the Problem]

In order to achieve the objects, the present invention provides an anticancer cathepsin formulation containing cathepsin E and/or an active fragment comprising or containing an active site thereof as an effective ingredient.

The present invention also provides an anticancer cathepsin formulation further containing an anticancer agent in addition to the above effective ingredient, which is capable of enhancing the action and effect of the anticancer agent. Further, the present invention provides a method for enhancing sensitivity of the anticancer agent by using the anticancer cathepsin formulation in combination with the anticancer agent.

Moreover, the present invention provides a method for cancer therapy by treatment of cancers with the above anticancer cathepsin formulation.
In addition, the present invention provides a tumor necrosis factor (TNF) homotrimer formed with a member or members of a TNF family.

In the present description, it is to be understood that the terms "anticancer cathepsin formulation" are intended to mean, in general terms, an anticancer cathepsin formulation containing cathepsin E and/or an active fragment comprising or containing an active site thereof as an effective ingredient as well as an anticancer cathepsin formulation further containing an anticancer agent in addition to the above effective ingredient. It is also to be understood that, even when the description is made with reference to either one of the anticancer cathepsin formulations, it is not restricted to the one and it can also be applied to the another one, too, in like manner, unless it is otherwise meant from the context. It is further understood that, in respect of the anticancer cathepsin formulation combined with the anticancer agent, the anticancer cathepsin formulation may be in the form of a single preparation in which the effective ingredient of cathepsin E is contained together with the anticancer agent or the anticancer cathepsin formulation may be in the form of separate preparations in which the effective ingredient of cathepsin E is contained separately from the anticancer agent and the separate formulations are administered in combined manner.

### BRIEF DESCRIPTION OF DRAWINGS

Fig. 1 is a view showing the effects of various kinds of cathepsins upon the survival of human prostate cancer cells ALVA-41.
Fig. 2 is a view showing the characteristics of cathepsin E-induced apoptosis in ALVA-41 cells.
Fig. 3 is an illustration showing the identification of TRAIL as an intermediate of cathepsin E-induced apoptosis in ALVA-41 cells.
Fig. 4 is an illustration showing sensitivity of various human prostate cancer cell lines to cathepsin E-induced apoptosis.
Fig. 5 is an illustration showing the effect of cathepsin E upon the growth of tumor formed by ALVA-41 cells in nude mouse injected with cathepsin E.
Fig. 6-1 is an illustration showing the direct connection of endogenous cathepsin E level with a decrease of growth and metastasis of mouse B16 melanoma cells in tumor.
Fig. 6-2 is an extension of Fig. 6-1.
Fig. 7 is a schematic view for the configuration of a plasmid of mouse cathepsin E transgene.
Fig. 8 is a view showing immunoblot analysis data of cell extracts of HEK 293 cells in which mouse cathepsin E is overexpressed.
Fig. 9 is an illustration showing the amount of expression of cathepsin E mRNA in various organs of transgenic mouse (CatE^{γg} mouse) with cathepsin E overexpressed and wild-type mouse (Wt) as a control.
Fig. 10 is an illustration showing the result of investigation of influences of cathepsins E and etoposide upon the survival of human leukemia cells.
Fig. 11 is an illustration showing the result of investigation of influences of a simultaneous administration of cathepsin E and etoposide and pre-treatment with etoposide upon the survival of human leukemia cells.
Fig. 12 is an illustration showing the result of investigation of influences of a simultaneous administration of cathepsin E and etoposide and a sole administration of etoposide upon the survival of U937 cells of human leukemia cells.

### BEST MODES FOR CARRYING OUT INVENTION

In the description which follows, a specific object such as a specific cancer or cancer cells will be described, however, it should be understood that the present invention is not intended at all to be limited to such specific objects and the description is solely for the purpose of illustration for explaining the contents of the invention in a more specific manner. In other words, although the following description is made regarding prostate cancer or prostate cancer cells, it is understood to be likely applied to cancers and cancer cells other than the prostate cancer and cells.

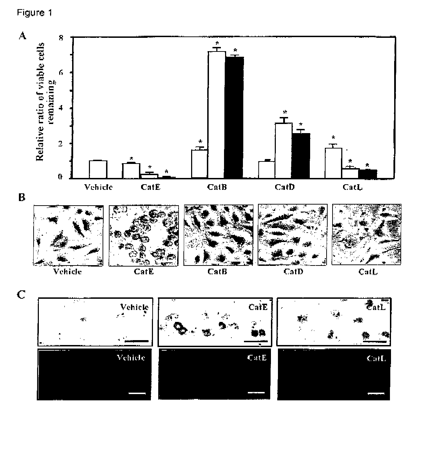

First, a description is made regarding the effects of various cathepsin enzymes on the survival of human prostate cancer ALVA-41 cells and apoptosis.
The cytotoxicity of each proteinase against cancer cells was investigated by in vitro incubating human prostate cancer ALVA-41 cells with cathepsin B, D, E or L for 20 hours at neutral pH range. As a result, it is found that cathepsin B and D increased the number of living cells in a concentration-dependent way (Fig. 1A). This result is consistent with the previous results that the cathepsins facilitate the growth, invasion and metastasis of tumor cells (Reference #7). Cathepsin L causes an increase in the number of these living cells in a low concentration (10 µg/ml), while it decreases the number of the living cells in a high concentration range (50-100 µg/ml). The previous observations are reported that the overexpression and secretion of procathepsin L increase the development of human melanoma cells into tumors (References #24 and #25). The deficiency of endogenous cathepsin L by antisense cDNA suppresses malignant phenotypes of glioma cells, melanoma cells, and osteosarcoma cells (References #25 and #26). This implies that this protein is involved in the growth and metastasis of tumors. On the other hand, cathepsin E decreases the number of survived carcinoma cells in a dose-dependent fashion. Morphologically, the cells treated with cathepsin E showed morphological features of apoptosis, including the cyst formation of cell membranes, regression of cytoplasms, formation of apoptotic bodies, and so on (Fig. 1B), white the cells treated with cathepsin L (100 µg/ml) show necrosis-like morphological changes such as, for example, swelling or decay of cells and nuclei, etc. In order to confirm a difference between patterns of cell death induced by cathepsin E and cathepsin L, the cells treated with such proteinases were analyzed by annexin V stain assay (for detection of the initial stage of apoptosis) and TUNEL assay (for detection of the late stage of apoptosis). As a result, the cells treated with cathepsin E (50 µg/ml) were found positive by staining with annexin V and TUNEL reagent while the cells treated with cathepsin L (100 µg/ml) were found negative by staining with both reagents (Fig. 1C). These results indicate that cathepsin E induces apoptosis of ALVA-41 cells and cathepsin L induces a cell death by necrosis. That the cell death of the cancer cells caused by cathepsin E is based on apoptosis is also confirmed from suppression by DEVD-fmlk acting as an inhibitor of caspase 3, 6 and 7 as well as z-VAD-fmlk acting as an inhibitor of caspase 1, 3, 4 and 7.

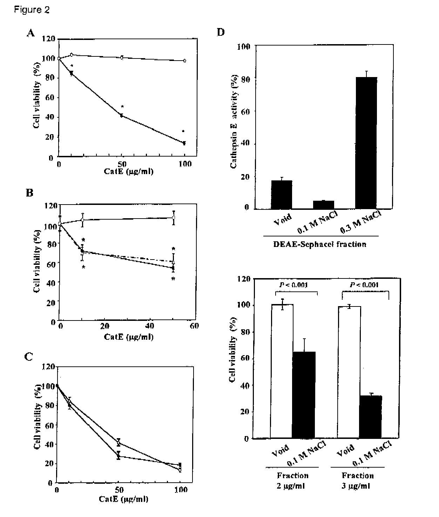

The cytotoxicity of cathepsin E against cancer cells is suppressed by addition of pepstatin A acting as an aspartic proteinase inhibitor to a culture medium of ALVA-41 cells (Fig, 1C). It is also found that an inactive cathepsin E mutant with two active sites of aspartic acid residues replaced with alanine (D98A/D283A.) did not show any effect upon the survival of human embryonic kidney cells (IIEK-293T cells) while the genetically engineered protein of the corresponding wild type induced apoptosis of HEK-193T cells (Fig. 2B) and ALVA-41 cells in a concentration-dependent fashion in the same manner as natural cathepsin E. These results indicate the ability of cathepsin E for the induction of apoptosis is thoroughly dependent upon its catalytic activity. Next, in order to investigate whether the ability of cathepsin E for inducing apoptosis is based on the direct action or the indirect action upon cancer cells, a culture supernatant of ALVA-41 cells treated with cathepsin E is added to a newly prepared culture medium of ALVA-41 cells in the presence or absence ofpepstatin A. It is found as a result that the cancer cells induced apoptosis in a dose-dependent fashion by the culture supernatant of the cells treated with cathepsin E (Fig. 2C). This indicates that cathepsin E-inducible apoptosis is induced by a certain molecule secreted from the surface of the tumor cells due to the proteinase action of cathepsin E. In order to investigate this further, the culture supernatant of the cells treated with cathepsin E was fractioned by DEAE-Sephacel chromatography. This result reveals that more than 80% ofthe cathepsin E activity was detected in a friction eluted with 0.3 M NaCl (References #27 and #28), however, the apoptosis-inducing activity was detected in a fraction eluted with 0.1 M NaCl, in which the cathepsin E activity was little detected.

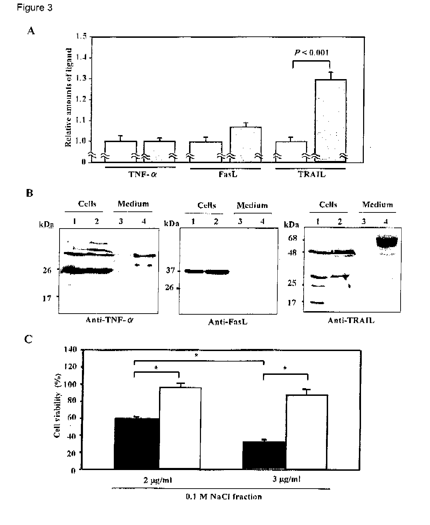

Then, a description will be made regarding the identification of TRAIL as a medium involved in apoptosis of tumor cells mediated by cathepsin E.
As described above, the mechanism of apoptosis of tumor cells is not yet understood well, however, the recent studies imply that apoptosis of tumor cells is suppressed by a cytokine molecule of a ligand type of a TNF family member such as, for example, TNE-α, FasL and TRAIL (Reference #1). The TNF family members secreted from the ALVA-41 cells treated with cathepsin E were investigated by ELISA assay using an antibody specific to each ligand. It is found from this result that TNE-α and FasL were little detected in the culture supernatant of the cells treated with cathepsin E while TRAIL was apparently secreted therefrom (Fig. 3C). It is known that TRAIL is connected to a receptor on the target cell surface by trimerization of its extracellular carboxyl terminal region, like other TNF family members, thereby transmitting apoptosis signals (References #29 - #32). Thus, the molecular type of a soluble TRAIL molecule contained in the culture supernatant of the ALVA-41 cells treated with cathepsin E was subjected to an immunoblot analysis using an anti-TRAIL antibody. As a result, there were detected two strongly immunoreactive bands of 63 kDa and 65 kDa as well as a minor band corresponding to 48 kDa (Fig. 3B). As immunoreactive bands of 17, 24, 30 and 48 kDa were detected in a cell extract before treatment with cathepsin E, the immunoreactive bands of 17 kDa and 24 kDA disappeared. Therefore, as the molecular weight of the extracellular portion of TRAIL is reported to be 21 kDa when calculated on the basis of its amino acid sequence (Reference #33), the proteins of 63 kDa and 65 kDa as well as 48 kDa, each detected in the cell culture medium treated with cathepsin E, are considered to be trimers as well as dimer of the soluble TRAIL, respectively. It was further confirmed that the results are consistent with ELISA data and no soluble trimer of TNF-α and FasL were produced in the culture supernatant of the ALVA-41 cells treated with cathepsin E.

Moreover, in order to investigate the degree to which this soluble TRAIL bears the activity of inducing apoptosis in the culture supernatant of the ALVA-41 cells treated with cathepsin E, the ability of inducing apoptosis of the 0.1 M NaCl fraction ofthe culture supernatant obtained by DEAE-Sephacel chromatography is investigated as to the degree to which it is lost by an anti-TRAIL antibody. It is found as a result that the effect of this fraction on the induction of apoptosis was lost to almost all extent (Fig. 3C). This made it apparent that the apoptosis activity of the tumor cells mediated by cathepsin E is totally dependent upon the trimer of TRAIL secreted from the cell surface.

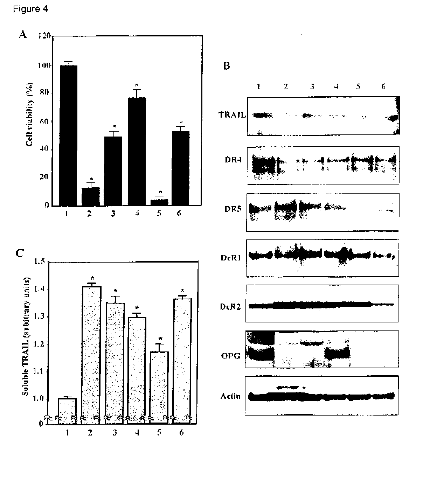

Now, a description will be made regarding the sensitivity of various human prostate cancer cell lines to cathepsin E-mediated TRAIL-dependent apoptosis.
As a result of investigations of various human prostate cancer cell lines about the sensitivity of cathepsin E to the TRAIL-dependent apoptosis, it was found that cathepsin E little exerted influences upon the survival and morphology of human prostate epithelium (PrE) cells, but it induced apoptosis for all cells (Fig. 4A). The sensitivity of the cathepsin E-induced apoptosis is increased by this order: PPC-1 <DU145 ALVA-41 <PC-3. Among these cells, the PC-3 cells show sensitivity to cathepsin E as to the cell survival effect higher by approximately 20 times than the PPC-1 cells.

It is reported that TRAIL facilitates or suppresses apoptosis by various acting receptors (Reference #29). For the human being, at least five kinds of receptors to TRAIL are identified. Among these receptors, TRAIL-R1(DR4) and TRAIL-R2(DR5) are death receptors capable of transmitting apoptosis signals (Reference #29). The remainder three kinds of receptors, i.e., TRAIL-R3(DcRl), TRA1L-R4(DcR2) and osteoprotegerin (OPG) as a soluble receptor, were identified as decoy receptors suppressing the apoptosis induced by TRAIL when overexpressed (Reference #29). As there is the possibility that the sensitivity of cells to apoptosis induced by TRAIL is dependent upon the relative expression level between the death receptor and the decoy receptors, the amount of expression of TRAIL and these receptors on the cell surfaces in various human prostate cancer cell lines and PrE cells are analyzed by cell surface biotinylation method and immunoblot analysis using an specific antibody corresponding thereto. All prostate cancer cell lines and 'PrE cells showed an amount of expression of TRAIL and a similar amount of expression of membrane receptors (two death receptors DR4 and DR5 as well as two decoy receptors Der1 and DeR2). Therefore, the expression of TRAIL and the membrane receptors made it apparent that it is not related directly to the difference of sensitivity between the cancer cells to the TRAIL-dependent apoptosis by cathepsin E (Fig. 4B).
On the other hand, the amount of the soluble decoy receptor OPG having no death domain and showing the suppression of formation of osteoclasts (References #34 and #35) is increased in the culture supernatant of PrE cells and PPC-1 cells much more than that of other cell lines used for the test. With the fact taken into consideration that the soluble decoy receptor OPG derived from the cancer cells is an important survival factor for a hormone-resistant prostate cancer cells and there is a negative relation between the endogenous OPG level and the ability ofapoptosis induction of the prostate cancer cells, it is considered that an increase of the amount of expression of the OPG may impart resistance to the TRAIL-dependent apoptosis for these cells by cathepsin. The amount of the soluble TRAIL trimers secreted by the treatment with cathepsin E is also varied with cancer cell lines (Fig. 4C). The amounts of the trimers are increased in this order: PrE <PC-3 <PPC-1 < ALVA-101 DU145 <ALVA-41 cells. As a significant difference in the amounts of expression of TRAIL is not recognized between various prostate cancer cell lines, it can be considered that the difference in the amount of production of the soluble TRAIL be based on the difference of efficiency of cutting the TRAIL on the cancer cell surfaces by cathepsin E. Therefore, an increase of the amount of expression of OPG or a decrease in efficiency of cutting off TRAIL by cathepsin E on the cell surface, or both, can partially explain a low sensitivity to the cathepsin E-induced apoptosis in the prostate cancer cell lines. However, a higher sensitivity of PC-3 cells to the cathepsin E-induced apoptosis cannot be explained by these two mechanisms, and this implies the presence of another determinative factor for determining sensitivity to this process, such as the presence of a mechanism causing a difference of the amount of expression of anti-apoptosis proteins, such as FLIP, JAPs, Bcl-xL, Bel-2, and so on.

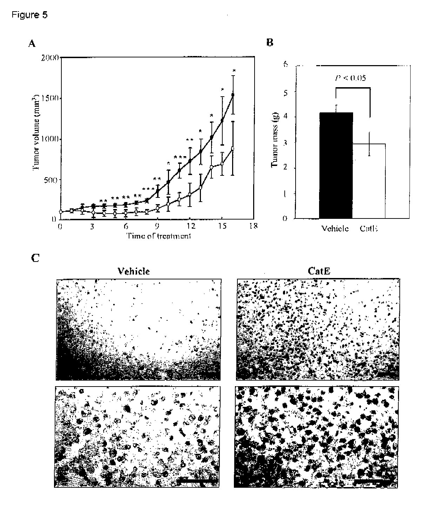

Next a description of the suppression of growth and metastasis oftumors in nude mice with human prostate cancer cells by the administration of cathepsin E.
It is investigated by in vivo research using nude mice with human prostate cancer cells transplanted subcutaneously as to whether the anticancer activity of cathepsin E observed in the culture system of cancer cells can be inspected. In this experiment, nude mice were transplanted subcutaneously with ALVA-41 cells and they were injected with purified cathepsin E (200 µg/kg) daily for 16 days at the central portion of the formed tumor when the volume of the tumor developed to approximately 100 mm³. As a control, physiological saline was injected in place of cathepsin E. The tumor cells administered with physiological saline have grown relatively gradually in the initial stage and thereafter increased exponentially (Fig. 5A). On the other hand, the tumor cells treated with cathepsin E have grown in a remarkably controlled manner as compared to the control. The effect of cathepsin E upon the regression of tumor was found to depend upon a dose of administration and it was remarkable at the daily dose of 200 µg/kg while the effect was rather decreased at the daily dose of 50 µg/kg and the effect of suppressing the tumor was little recognized at the daily dose of 30 µg/kg or lower. At the time when the tumor volume reached approximately 400 mm³, cathepsin E was administered at a rate of 400 µg/kg once per day. After the administration was continued for 10 days, the tumor cells were removed and the weight of the tumor cells was measured. As a result, it was found that the weight of the tumor was significantly lower in the group of administration with cathepsin E than the control group (Fig. 5B). The tumor cells of each group were stained with TUNEL to compare the number of the tumor cells that caused apoptosis, and it was found that a number of the apoptosis cells were observed in the group of treatment with cathepsin E while the apoptosis cells were little detected in the control group (Fig. 5C). This indicates that cathepsin E administered from the outside can induce apoptosis in cancer cells in vivo at an individual level. As the TRAIL-dependent apoptosis of the cancer cells by cathepsin E can be enhanced by increasing a number of administration or an amount of administration, a greater effect upon regression of tumor cells can be expected by improving the method of administration of cathepsin E. Moreover, no effect upon toxicity toward normal tissues and cells can be recognized at all by the administration of cathepsin E.

The following is a description of the relationship of a level of expression of endogenous cathepsin E with the effect upon suppression of the growth of tumors.
It is known that tumor-infiltrating effecter cells such as lymphocytes, natural killer cells, monocytes, dendritic cells and so on play a significant role in the TRAIL-dependent suppression of development or metastasis of tumors (References #36 and #39). As cathepsin E is expressed predominantly in cells of the immune system including, for example, lymphocytes, macrophages, dendritic cells, microglia and so on (References #9, #10 and #40), the cathepsin E in these cells is considered to contribute to the host defense mechanism for tumor cells. Therefore, in the present invention, the influence of the amount of expression of cathepsin E upon the growth, metastasis and apoptosis of tumors was reviewed for three types of inbred mice showing different levels of cathepsin E expression (transgenic mice (i.e., CatE^{-/-}, CatE^{Tg} overexpressing cathepsin E, and their wild-type syngenic mice (Wt)). Mice of each genotype were injected subcutaneously with inbred mouse melanoma B16 cells and the sizes of tumors formed were observed until day 21. It was observed that the growth of tumor in each animal group was relatively slow up to day 8 and thereafter increased gradually. During the period from 10 to 15 days after injection, the growth of tumors in CatE^{-/-} mice was significantly higher than CatE^{Tg} and Wt mice, however, no significant difference was any longer shown between the CatE^{-/-} and Wt mice in day 19 after injection (Fig. 6A). It was further observed that the growth of tumor in the CatE^{Tg} mice was controlled to an extent stronger than the other two inbred mice groups during the total period of experimentation and the weight of tumor of the CatE^{Tg} mice at day 21, i.e., the last day of experimentation, was lighter by approximately one thirds and by approximately one fifth than the CatE^{-/-} and Wt mice, respectively. A 56-day mortality rate of mice injected with B16 melanoma cells was such that the mortality rate ofthe CatE^{-/-} mice was highest, that of the Wt mice was next high, and that of the CatE^{Tg} was lowest. In other words, the survival rate of the CatE^{-/-} mice was 20%, while that of the wild-type mice was 60% and that of the CatE^{Tg} mice was 80% (Fig. 6B). These results indicate that there is a reverse correlation between the propagation of tumor and the amount of expression of cathepsin E.

In order to investigate whether a decrease in the mortality rate of the CatE^{Tg} mice is due to acceleration or exclusion of apoptosis of tumor cells, a degree of expression of apoptosis tumor cells was compared among groups of each genotype mouse. A TUNEL assay of the tumor cells collected from each animal group at the day 23 after inoculation revealed that the apoptosis cells were remarkably increased in the CatE^{Tg} mice, however, the apoptosis cells were very slight in the CatE^{-/-} and Wt mice (Fig. 6C). What is to pay attention herein is that the melanoma B16 cell used therein is resistant to TRAIL-dependent apoptosis (References #39 and #42). Therefore, it is considered that the apoptosis of the cancer cells observed in the CatE^{Tg} mice be induced by a mechanism different from the mechanism mediated by TRAIL. A morphological observation indicates that the tumor sites in all mouse groups inoculated with melanoma B16 cells were infiltrated with a large number of effecter cells such as lymphocytes, macrophages and so on. It was found, however, that an immunological staining using antibodies to F4/80 antigen and MHC class II molecule showed the highest number and activation degree of macrophages in the tumor cells of the CatE^{Tg} mice while it showed the next highest number and activation degree of macrophages in those of the WT mice and extremely low in those of the CatE^{-/-} mice (Fig. 6D). These data are consistent with the previous results that the number of macrophages in an exudate from patients with cancer was reversely proportional to malignancy of cancer (References #43 and #44). It is known that macrophages and lymphocytes demonstrate cytotoxicity through the production of active oxygen species or reactive nitrogen intermediates and effecter molecules such as IFN-α, IFN-γ, IL-1, IL-12 and so on, in addition to the effect of induction of TRAIL-dependent apoptosis (Reference #45). Moreover, it becomes apparent that the activated macrophage injures the tumor cells effectively through an antibody-dependent mechanism and a non-antibody-dependent mechanism. It is considered, accordingly, that the suppression of growth of tumor in the CatE^{Tg} mice, which is caused to occur concurrently with apoptosis of the TRAIL-resistant B16 cells, and a decrease in the mortality of these animals are considered to be caused by cytotoxicity of the tumor-infiltrating effecter cells, particularly activated microphagcs.

It can further be noted that a number of dead cells and a region of necrosis of portion around tumor containing those dead cells increased remarkably in the following order: CatE^{Tg} mice < Wt mice < CatE^{-/-} mice (Fig. 6E). This is inversely proportional to a distribution ofblood vessels in the necrosis region and indicates that the exclusion of the dead cells in the circulatory system and the infiltration of inflammatory cells into the tumor site are highest in the CatE^{Tg} mice and higher in the Wt mice than the CatE^{-/-} mice. It was moreover found that the structure of vascular endothelial cells observed at the central and surrounding portions of tumor was damaged to a remarkable extent in the CatE^{-/-} mice compared with the CatE^{Tg} and Wt mice (as shown in the lower panel of Fig. 6E). Such failures of the vascular system of the CatE^{-/-} mouse are considered to facilitate a leakage of blood contents such as erythrocytes and so on outside the blood vessels. With the fact taken into consideration that tumor-infiltrating macrophages induce fresh blood vessels in the region with no blood vessels and play an important role in remodeling the vascular system once formed, the data of the present invention implies that cathepsin E controls the vascular system in the tumor site by the aid of activation of the tumor-infiltrating macrophages.

It was further found that the level of expression of the endogenous cathepsin E exerts an influence upon the degree of metastasis of cancer cells. As a result of experimentation carried out by intravenously injecting groups of mice of each genotype with melanoma B16 cells and measurement for the number of colonies of the cancer cells transferred to the lungs at day 23 after injection, it was found that the number of colonies in the CatE^{-/-} mice was larger by approximately two times and eight times than the Wt mice and the CatE^{Tg} mice, respectively. This indicates that the deficiency of cathepsin E facilitates the metastasis of the cancer cells to the lungs. Therefore, these data imply that the endogenous cathepsin E suppresses the metastasis of the cancer cells.

Now, a brief description will be made regarding TRAIL. The extracellular terminal portion COOH of TRAIL is cut off and separated from the surface of the cell, like other members of the TNF family. The soluble TRAIL thus separated therefrom forms a homotrimer inducing apoptosis in various cancer cells (References #2 and #3). It can be noted herein, however, that the soluble TRAIL little induces apoptosis in normal cells. This noticeable property of TRAIL implies utility in cancer therapy as an agent for treating human cancers. In fact, an agonist (including an action-type antibody) for a recombinant soluble TRAIL and a TRAIL receptor is now being subjected to phase I clinical trials as a new anticancer agent in the US. Therefore, it is extremely important to determine a proteinase involved in the production of soluble TRAIL from the cell surface of various cell lines. At the present time, it is known that TNF-α and FasL are separated by TNF-α converting enzyme (TACE) (References #47 and #48) and MMP-7 (References #49, #50 and #51), however, a proteinase involved in cutting TRAIL is not determined yet. The observations so far made implies that a cellular cysteine proteinase may be involved in the separation of TRAIL because the expression of TRAIL on the cell surface of Sf9 cells expressing a full-length engineered TRAIL is enhanced by a cysteine proteinase inhibitor such as leupeptin and E-64, but it is not enhanced by a metalloproteinase, and further the separation of a soluble TRAIL of 20 kDa from the cell extract is inhibited by the cysteine proteinase inhibitor (Reference #52). It is not clarified yet, however, that these observations so far obtained under in vitro experimentation conditions represents an event of physiological decomposition of proteins undergoing the production of the soluble TRAIL actually in the living body.

The present inventors made it apparent by the present invention for the first time that cathepsin E is an enzyme responsible for induction of apoptosis in cancer cells only without exerting any influence upon normal PrE cells, by cutting off the TRAIL from the surfaces of human prostate cancer cells and forming a soluble trimeric molecule. Cathepsin E can separate the soluble TRAIL from all human prostate cancer cells, however, it is recognized that the efficiency of induction of apoptosis is varied among cancer cell lines. Some cell lines such as PPC-1 and DUI45 cells are relatively resistant to apoptosis inducible by cathepsin E, like normal PrE cells, but other cell lines such as PC-3 and ALVA-41 cells show a high sensitivity. As there is no substantial difference in levels of expression of TRAIL, DR4, DR5, DcR and DcR2 on the cell surface among these cell lines, it is implied that the difference of the amounts of expression of these molecules is unrelated to the difference of sensitivity to cathepsin E-inducible apoptosis among the cancer cells. Rather, it can be noted that an increase in the production of soluble decoy receptor OPG may be one factor that provides PrE cells and PPC-1 cells with resistance to cathepsin E-inducible apoptosis. Moreover, the high sensitivity of the ALVA-41 cells to cathepsin E can be explained from a relatively great amount of the soluble TRAIL discharged from these cell surfaces. However, the high sensitivity of the PC-3 cells to cathepsin E cannot be explained by the amounts of OPG and the soluble TRAIL only so that there is considered to be another factor because the expression of an anti-apoptosis protein such as FLIP, IAPs, Bel-x_{L}, Bcl-2 and so on is lower in the PC-3 cells than in the other cell lines.

It is found that the results of in vitro experimentation to investigate the suppression of growth of tumor cells and induction of apoptosis by cathepsin E are consistent well with the results of research made at an individual level using nude mice transplanted with human cancer cells. A daily administration of cathepsin E to nude mice transplanted with human prostate cancer cells suppressed the growth of the tumor cells and induced apoptosis without exerting any morphological influences at all upon normal tissues and cells. These results are consistent with the observation that the survival life of mice inoculated with the cancer cells was extended for a considerable long period. It is further considered that, from the matter of fact that apoptosis of a majority of the tumor cells is observed at the last day of administration of cathepsin E, the regression of tumor can be effectively induced by administering cathepsin E more frequently or increasing an amount of administration of cathepsin E. With the fact taken into consideration that TRAIL is produced by the tumor effecter cells such as activated T cells (References #39 and #53), B cells (Reference #54), natural killer cells (Reference #55), dendritic cells (Reference #39), monocytes (Reference #37) and so on, it can be assumed that the production of the soluble TRAIL from cells of the immune system can also induce apoptosis and regression of tumor cells.

It is also found that cathepsin E can demonstrate an activity of killing tumor through a mechanism independent from TRAIL. Although a very large number of tumor cells are sensitive to TRAIL-dependent apoptosis, a certain tumor cell line rather shows a resistance to the TRAIL-dependent apoptosis (References #38 and #41). The observations obtained by the present invention indicate that CatE^{Tg} mice transplanted subcutaneously with B16 melanoma cells demonstrated a number and a degree of activity of the tumor-infiltrating effecter cells, particularly activated macrophages, considerably greater than inbred CatE^{-/-} mice and Wt mice. It is considered that, with the fact taken into consideration that the activated macrophage infiltrating into the tumor site has the ability of attacking and excluding the cancer cells due to a direct contact with the cancer cells or an indirectly mutual action through a humoral factor such as a cytokine or the like (Reference #36), the tumor-infiltrating activated macrophage can induce apoptosis of the tumor cells together with other cells of the immune system by means of a mechanism separate from the mechanism of the TRAIL-dependent apoptosis. In connection with this point, it is reported that a degree of the presence of the tumor-infiltrating activated macrophage is closely related to a good prognosis of patients with cancer (Reference #36). The activation of macrophage by IFN-γ and LPS indicates an increase in expression of cathepsin E at a mRNA level and a protein level as well as an increase in secretion of cathepsin E (Reference #12), and the deficiency of cathepsin E causes a functional failure of macrophage (Reference #19). These observations indicate a close relation of cathepsin E with the function of the activated macrophage. Therefore, this observation that in the subcutaneous tumor of the CatE^{-/-} mice transplanted with B16 melanoma cells, the number of the infiltrating macrophages and the active state (as reflected by the expression of MHC class II molecule) arc both decreased apparently, as compared with the CatE^{Tg} mice and the Wt mice, indicates that the level of expression of cathepsin E in the macrophage is strongly related to the suppression of the growth of tumor and an individual death. Therefore, cathepsin E is considered to suppress the propagation and growth of B16 melanoma cells by the aid of the activity of cytotoxicity of the tumor-infiltrating activated macrophage.

What is to be noted herein is that the tumor-infiltrating macrophages in tumors have two mutually competing actions, that is, the action of regressing tumor cells and the action of facilitating tumor. For instance, the tumor-infiltrating macrophage of a low concentration has the effect the lysis of cells, while the tumor-infiltrating macrophage of a high concentration has the effect of facilitating the growth of tumor (References #56 and #57). It is considered that macrophage has useful anticancer effects through versatile functions (for example, a risen phagocytosis, an attraction and activation of T lymphocytes or neutrophils, an activation of natural immune or acquired immune, etc.) (Reference #58), while it facilitates the growth and metastasis of tumor through some mechanisms (for example, facilitation of migration and invasion of tumor cells, a rise of decomposition and alteration of an extracellular matrix, facilitation of angiogenesis, etc.) (Reference #59). These competing functions are considered to result from qualitative and quantitative diversity of various cytokines and active oxygen species produced by macrophage. In the present invention, the present inventors have clarified the facts for the first time that the CatE^{Tg} mice suppress remarkably the subcutaneous growth and metastasis of mouse B16 melanoma cells to the lungs, resulting in a decrease of the mortality rate as compared with the inbred CatE^{-/-} mouse and Wt mouse and that the tumor of mouse B16 melanoma cells has a clear region for an apoptotic necrosis. As described above, that the level of expression of the endogenous cathepsin E is positively correlated to the suppression of growth and metastasis of cancer of the B16 melanoma cells and to an increase and activation of the tumor-infiltrating macrophage implies that cathepsin E can exclude the tumor cells through the cytotoxicity of macrophage, ability of induction of apoptosis, and the regression of the microvasculature of tumor. Therefore, cathepsin E is considered to destruct the cancer cells by at least two mechanisms, that is, the mechanism of the TRAIL-dependent apoptosis and the mechanism of tumor-infiltrating macrophage-inducible cytotoxicity. The activity of decomposition of proteins of many proteinases such as cathepsin B, L and D as well as MMPs forms a microenvironment favorable for facilitating the growth of tumor, but a proteinase inducing the suppression of tumor growth and metastasis is little known. In this respect, cathepsin E indicates an example of a useful proteinase having an anticancer action.

A common problem that almost all anticancer agents cannot conquer yet is a problem with their severe side effects. Therefore, the strategy effective for screening anticancer agents places a target on the induction of apoptosis to target tumor cells only without causing any cytotoxicity whatsoever for normal cells. The observations so far made imply that an administration of an agent capable of increasing the expression of cathepsin E or endogenous cathepsin E can become a promising strategy for an anticancer therapy. Therefore, the anticancer therapy using cathepsin E as a basis can be applied to treatment of human cancers in situ when cathepsin E is administered solely or in combination with another therapy.

Therefore, a main embodiment of the present invention is to provide an anticancer cathepsin formulation. The anticancer cathepsin formulation is a cathepsin formulation containing cathepsin E and/or an active fragment comprising its active site or containing its active site as an effective ingredient and a cathepsin formulation further containing an anticancer agent, in addition to the above effective ingredient. The latter cathepsin formulation includes a preparation in the form of a formulation in which the above ingredient and the anticancer agent are formulated together and administered simultaneously and a preparation in the form of a formulation in which the above ingredient and the anticancer agent are separately formulated and administered.

The anticancer cathepsin formulation according to the present invention is characterized in containing cathepsin E and/or an active fragment comprising its active site or containing its active site.
Cathepsin E to be used for the present invention has a sequence of bases as represented by SEQ ID #1. The active site of the cathepsin E of the present invention is a range containing aspartic acid at positions 98 and 283. In the present invention, the active fragment comprising the active site of cathepsin E means an active fragment comprising a region containing aspartic acid at positions 98 and 283. The active fragment containing the active site of cathepsin E also means an active fragment containing a region having aspartic acid at positions 98 and 983 as a part thereof.

In the description, the terms "cathepsin E" is understood to be used in the meaning encompassing its active fragment unless they can be interpreted in a different meaning apparently from the context or unless otherwise stated, when the terms "cathepsin E" are described solely.

In the present invention, cathepsin E has the properties ofreleasing and activating TRAIL particularly for many kinds of tumor cells with TRAIL present on their surfaces leading to suppression of the growth and metastasis of the tumor cells and inducing apoptosis. Further, surprisingly, it is found that cathepsin E launches an attack on the tumor cells alone without adversely affecting the normal cells whatsoever. Therefore, it can be expected that the cathepsin formulation ofthe present invention containing cathepsin E as an active ingredient can be an extremely effective anticancer agent.

As described above, a very large number of tumor cells are sensitive to TRAIL-induced apoptosis, however, cathepsin E can also demonstrate the activity of suppressing tumor through a mechanism independent from the apoptosis mechanism by the aid of TRAIL. In other words, cathepsin E to be used for the present invention can demonstrate the activity of suppressing tumors of tumor cell lines, for example B16 melanoma cells, etc., too, which have rather a resistance to the TRAIL-dependent apoptosis.

The cathepsin formulation according to the present invention can also be used in combination with other anticancer agents, that is, for a so-called combination therapy. As the anticancer agents to be used for the combination therapy, there may be used any anticancer agent as long as it can enhance or add the effects or activities of cathepsin E, and the anticancer agent is not limited to a particular one. In the event where the cathepsin formulation of the present invention is combined with the anticancer cathepsin formulation, it can be expected that the therapy effect can be achieved at a lower dose or doses of the anticancer agent than when it is administered solely. Therefore, it can be expected that, when the anticancer agent is administered together with cathepsin E, the side effects ofthe anticancer agent caused otherwise to occur by the anticancer agent can be suppressed or minimized.

It is to be understood in this description that, even when the terms "anticancer cathepsin formulation" are used instant, they are intended to mean an anticancer cathepsin formulation containing an anticancer agent in combination with cathepsin E as well as the cathepsin formulation containing cathepsin E as a sole active ingredient, unless it should be interpreted in a different meaning from the context or unless otherwise stated herein.

It is further to be understood that the combination therapy in accordance with the present invention encompasses, within the scope of invention, the formulation containing cathepsin E and the anticancer agent or anticancer agents in an integrated form and the formulation by which the cathepsin E and the anticancer agent or anticancer agents are administered separately to enhance or supplement their mutual actions.

The anticancer agent to be combined with the cathepsin formulation according to the present invention may include, but not be limited to, cytotoxic anticancer agents such as alkylating agents, antimetabolites, antibiotics, microtubule-disrupting agents, etc. and molecular target therapy agents.
As the alkylating agents, there may be mentioned, for example, nitrogen mustards, e.g., cyclophosphamide, ifosfamide, melphalan, mechlorethamine, chlorambucil, etc., alkyl sulfonates, e.g., busulfan, improsulfan tosilate, etc., ethylene imines, e.g., thiotepa, etc., methylmelamines, e.g., hexamethylmelamine, etc., nitroureas, e.g., carmustine, nimustine, ranimustine, lomustine, streptozocin, etc., triazenes, e.g., dacarbazine, etc.

As the antimetabolites, there may be mentioned, for example, folic acid analogues, e.g., methotrexate, etc., pyrimidine analogues, e.g., 5-fluorouracil (5-FU), tegafur, UFT, doxifluridine (5'-DFUR), carmofur, capecitabine, cytarabine, floxuridine, cytarabine ocfosfate, enocitabine, etc., purine analogues, e.g., mercaptopurine, 6-mercaptopurine riboside, thioguanine, pentostatin, etc.

The anticancer antibiotics may include, but not be limited to, actinomycin D, bleomycin, peplomycin, mitomycin C, aclarubicin, streptozocin, daunorubicin, doxorubicin, pirarubicin, epirubicin, neocarzinostatin, zinostatin stimalamer, idarubicin, etc. As the anticancer agents of plant alkaloid type, there may be mentioned, for example, vinca alkaloids, e.g., vincristine, vinblastine, etc., epipodophyllotoxines, e.g., etoposide, teniposide, etc., taxols, e.g., paclitaxel, docetaxel, etc., vinorelbine, vindesine, irinotecan, sobuzoxane, epothilone, desoxycpothilone, and so on.

The platinum-complex compounds may include, but not be limited to, cisplatin, carboplatin, nedaplatin, and so on. As anticancer agents which may be classified into other categories, there may be mentioned, for example, anthracenediones, e.g., mitoxantrone, etc., methylhydrozines, e.g., procarbazine, etc., vitamin A metabolites, e.g., tretinoin, etc., dacarbazine, hydroxycarbamide, pentostatin, L-asparaginase, and so on.

Among the anticancer agents, for example, the alkylating agents, antimetabolites and platinum complexes are agents for controlling the S phase of the cell cycle, during which DNA is synthesized. On the other hand, the antibiotics and the plant alkaloids such as paclitaxel and docetaxel have the activity for suppressing the formation of microtubules and they are agents for mainly controlling the M phase of a course of the cytokanesis. Therefore, these anticancer agents can be used solely or in combination therewith. Where they are used in a combined way, for example, a combination of the antimetabolite with the platinum-complex compound, in particular a combination of cisplatin (CDDP) with 5-fluorouracil (5-FU), is preferred.

The anticancer cathepsin formulation according to the present invention can be applied to treatment of various cancers. The cancers to which the cathepsin formulation of the present invention can be applied may include, but not be limited to, cancers of the esophagus, throat cancer, thyroid, stomach, liver, lungs (including vesicles), breast, pancreas, gallbladder, kidney, large intestine, rectum, colon, bladder, prostate gland, ovary, skin (including squamous cell carcinoma), etc.; hematopoietic leukemias ofthe lymphatic lineage, e.g., lymphocytic leukemia, lymphoblastic leukemia, cell leukemia, Hodgkin's lymphoma, Non-Hodgkin's lymphoma, etc.; hematopoietic leukemias of the myelogenous lineage, e.g., myelogenous leukemia, myelodysplastic syndromes, promyelocytic leukemia, etc.; sarcomas of the central and peripheral nervous systems, e.g., astrocytoma, neuroblastoma, glioma, schwannoma, etc., sarcomas of the mesenchymal system, e.g., fibrosarcoma, rhabdomyosarcoma, osteosarcoma, etc., other cancers, e.g., melanomas, xeroderma pigmentosum, keratinizing epithelioma, seminoma, thyroid folliculoma, tertoma, and so on.

The cathepsin formulation according to the present invention may contain pharmacologically acceptable carriers and additives including, but not being limited to, diluents, excipients, stabilizers, buffers, coloring agents, and son. They may include, but not be limited to, sodium acetate, sodium lactate, sodium chloride, potassium chloride, calcium chloride, sorbitan monolaurate, triethanolamine oleate, and so on.

The anticancer cathepsin formulation according to the present invention may be administered through oral routes or non-oral routes such as intravenous injection, intramuscular injection, intraperitoneal injection, transdermal injection, rectal administration, local administration, and so on. For example, tablets, capsules, aqueous solutions and suspensions may be administered orally. The solid preparations for administration through oral route may include, for example, usual carriers such as lactose, corn starch, magnesium stearate and so on, and the liquid preparations for administration through oral route may include, for example, emulsifiers or emulsifiers.

In the present invention, the dose of cathepsin E to be used in combination with the anticancer agent may not be limited to a particular one and be varied in a wide range according to the predetermined dose of the anticancer agent, as long as the anticancer activity (including the apoptosis-inducing activity) of the anticancer agent can be enhanced. In fact, as the combination administration of cathepsin E can enhance sensitivity of the anticancer agent, the anticancer agent can be administered at a dose lower than the predetermined dose of the anticancer agent, In general, a dose (a unit dose) of cathepsin E per administration may be varied with a kind and a form of administration of the anticancer agent to be used in combination, a kind of the tumor, a body weight and condition of a patient, and a disease condition. The dose of cathepsin E may be varied from 1 ng/kg to 200 mg/kg of body weight for subcutaneous administration, from 0.005 mg/m² to 600 mg/m² of body surface for subcutaneous administration, and from 2.5 mg/kg to 2 g/kg of body weight for oral administration.

The following is a specific description of the present invention by way of examples. It is to be understood herein, however, that the present invention is not limited at all to the following examples.

### [Example 1]

### (Measurement for the survival rate of cells)

The survival rate of cells was measured by Cell Counting Kit-8 in accordance with a manufacturer's manual. The method will be described briefly as follows. A 96-well (each 100 *µ*1) plate was inoculated with cells (1 x 10⁴ cells) and incubated for 24 hours, and the cells were incubated in a non-serum culture medium Opti-MEM together with a given concentration of each cathepsin purified from rat stomach (References #27 and #60-#62), Thereafter, the kit reagent (10 µl) was added to each well and the wells were incubated for another 1 hour. After incubation, each well was measured at the absorbance of 450 nm with a microplate reader.

### (Measurement for apoptosis)

The cells were measured for a morphological variation of apoptosis by phase contrast microscopy. The cells of prophase and post-phase apoptosis were detected by annexin V staining and TUNEL assay. In order to carry out the annexin V staining of ALVA-41 cells, the cells were exposed to cathepsin E or cathepsin L for 4 hours and centrifuged. After centrifugation, the separated cells were washed twice with PBS and incubated with fluorescein isothiocyanate-conjugated annexin V for 10 minutes at room temperature in a dark place. The cells that were positive as a result of the annexin V staining were then detected with fluorescence microscope. The TUNEL assay was carried out for the incubated tumor cells using Apop Tag Peroxidase Apoptosis Detection Kit and for heterografts using Tumor TACS In Situ Apoptosis Detection Kit.

### (Preparation of recombined cathepsin protein)

A recombinant wild-type protein and its mutant protein (D98A/D283A) with two aspartic acid residues of its active site replaced by alanine were expressed in HEK 293T cells and purified in substantially the same manner as described above (Reference #63).

### (DEAE-Sephacel chromatography and immunodepletion

ALVA-41 cells (9 x 10⁹ cells) were incubated in non-serum culture medium Opti-MEM with cathepsin E (100 µg/ml) at 37°C for 20 hours and then centrifuged at 20,000 x g at 4°C for 30 minutes. The resulting supernatant was concentrated and centrifuged, and the resultant supernatant was dialyzed with 10 mM sodium phosphate buffer (pH 7.0) containing 0.05% Brij 35. Then, the dialysate was added to a DAEA-Sephacel column equalized with the same solution, and then eluted stepwise with 0.01M and 03M NaCl. The cytotoxic activity and cathepsin E activity of the column fractions were investigated using newly prepared ALVA-41 cells and a cathepsin E-specifie substrate, i.e.,
MOCAc-Gly-Ser-Pro-Ala-Phe-Leu-Ala-Lys(Dnp)-D-Arg-NH₂ (Reference #64). For the immunodepletion of TRAIL from the 0.01M NaCl fraction, the cells were incubated first with a mouse monoclonal antibody to TRAIL or a mouse immunoglobulin G (each 1 µg/ml) as a control and then with protein G-Sepharose 4 Fast Flow overnight at 4°C. Thereafter, the resin was removed by centrifugation and the resulting supernatant was assessed for the effect upon the survival of the ALVA-41 cells.

### (SDS-PAGE and Immunoblot Analysis)

The SDS-PAGE and immunoblot analyses were carried out according to prior art literature (Reference #63). The protein fractioned by SDS-PAGE was migrated on nitrocellulose membrane and was blocked with 5% non-fat dried milk. Then, the nitrocellulose membrane was incubated overnight at 4°C with a primary antibody, i.e., anti-TRAIL antibody (1/300-fold dilution), anti-TNF-α antibody (1/200-fold dilution), anti-FasL antibody (1/500-fold dilution), anti-DR4 antibody (1/500-fold dilution), anti-DR5 antibody (1/100-fold dilution), anti-DcR1 antibody (1/500-fold dilution), anti-DcR2 antibody (1/500-fold dilution), anti-osteoprotegrin (1/150-fold dilution) or anti-β-actin antibody (1/500-fold dilution). The resulting membrane was washed and then incubated together with horseradish peroxide-conjugated rabbit, mouse or goat immunoglobulin G antibody (1/4000-fold dilution) as a secondary antibody at 4°C overnight. The resulting membrane was then washed several times and an immunocomplex was detected with a chemiluminescent reagent. The measurement for density was measured with LAS 1000 analyzer.

### (Biotinylation of Cell Surfaces)

The biotinylation of cell surfaces was carried out in a manner as will be described below. The prostate gland cells were placed in a dish in a ∼80% confluent state, washed twice with ice-cold PBS, and then labeled with biotin by incubation with sulfo-NFS-S-SS-biotin, i.e., a cell-nonpermeable and decomposable reagent, at 4°C for 30 minutes. The non-conjugated biotin was quenched by washing the cells six times with a 100 mM glycine-PBS solution, and the cells were lyzed with 1% Triton X-100 and 200 µl of 100 mM phosphate buffer (pH 8.0) containing 1 mg/ml of phenylmethylsulfonyl fluoride, 1 mg/ml of leupeptin, 1 mg/ml of pepstatin and 1 mg/ml of aprotinin. The resulting vell lysate was centrifuged at 20,000 x g at 4°C for 20 minutes and the resulting supernatant was gradually stirred together with streptoavidin-agarose beads at 4°C for 2 hours. The beads so treated were washed twice with a solution containing 10 mM Tris-HCl (pH 7.5), 0.1% SDS, 0.1% Triton X-100, 2mM EDTA and 1mM NaN₃, twice with a solution prepared by ading 1 M NaCl and 0.1% sodium lauroyl sarcosinate to the above solution, and twice with 5mM Tris-HCl (pH 7.0). Then, the resulting beads were incubated in a SDS sample buffer at 100°C for 5 minutes and eluted, followed by subjecting the eluted protein to SDS-PAGE and immunoblot analysis.

### (ELISA of TRAIL, FasL and TNF-α)

ALVA-41 cells (1x10⁶ cells per a 6-cm dish) were incubated together with cathepsin E (100 µg/ml) in a non-serum culture medium Opti-MEM at 37 °C for 20 hours. The resulting culture supernatant was measured for amounts of TRAIL, FasL and TNF-α with ELSA kit in a manner as will be described briefly. A sample and a standard product (each 100 µl) were added to a microtiter plate immobilized with a monoclonal antibody to the corresponding member of the TNF family and incubated at room temperature for 2 hours. Then, the wells of the microtiter plate were washed four times with a washing buffer and 100 µl ofbiotin-conjugated monoclonal antibody was added to each wells ofthe plate, following by incubation at room temperature for 1 hour and washing the wells of the plate completely. Then, they were incubated together with 100 µl of streptoavidin-conjugated horseradish peroxidase at room temperature for 30 minutes and washed four times with a washing buffer. Stabilized chromogen (100 µl) was added to each well of the plate which was in turn incubated in a dark place at room temperature for 30 minutes. After an addition of a reaction terminating solution, the wells were measured at 450 nm with microreader.

### (Immunohistochemistry)

For a graft fixed to formalin and embedded in paraffin, F4/80 and MHC class II molecule were investigated using CHEMICON IHC Select(R) Immunoperoxidase Secondary Detection System. Paraffin was removed with xylene and alcohol from a slide which was in turn treated with 3% hydrogen peroxide for 10 minutes. After rinsing, the graft was incubated with a blocking reagent (normal goat serum) and rinsed. The graft was then incubated with anti-F4/80 antibody (1/500-fold dilution) or anti-MHC class II molecule antibody (1/500-fold dilution) as a primary antibody at room temperature for 10 minutes and then with IHC select(R) biotinylated secondary antibody goat anti-rat IgG as a secondary antibody at room temperature for 10 minutes. The graft was then treated with streptoavidin-conjugated HRP at room temperature for 10 minutes and subjected to reaction with a chromogen reagent (3,3'-diaminobenzidine) at room temperature for 10 minutes. The fragment so treated was subjected to reverse staining with hematoxylin and investigated with optical microscope.

### (Experimental animals)

All experimental animals were bred in a sterile facility in accordance with guidelines of The Japanese Pharmacological Society. The animal experiments were carried out under the approval of The Experimental Animal Commission of Kyushu University Dental Research Laboratories. A wild-type mouse as a C57BL/6 gene background and a CatE^{-/-} mouse were used in accordance with the manner as described on prior art (Reference #17). In order to generate a transgenic mouse overexpressing cathepsin E, a mouse cathepsin E transgene labeled with hemagglutinin (HA) was constructed. A total RNA was extracted from mouse spleen with a TRIsol reagent and a first-strand cDNA was synthesized with a reverse transcriptase. Using the first-strand cDNA as a template, cathepsin E was amplified with EX tag polymerase using primers having the following sequences:
5'-CGGTCTAGAGAGATCGGAGCAGAGTGAGAG-3'; and
5'-GGACCCGGGTAACAGGYTTAAATGGGTATCA-3'
where the underlined sequences represent Xba I and Sma I sites, respectively.
The amplified fragment was subcloned on pBluescript SK with a HA label, and site-directed mutation was induced in order to remove a gap between mouse cathepsin E (mCE) and HA. After digestion with Xba I and filling with Klenow fragment. Thereafter, pXhoI linker was added at both ends of the fragment, and the resulting plasmid was digested with Xho I, followed by connection to pCAGGS treated with bovine alkaline phosphatase. A transgenic mouse was then formed with the resulting plasmid. The linker DNA fragment of the target vector was microinjected into the oosperms of C578L/6J mice. A hemizygote Tg mouse was formed by mating the founder mouse with a wild-type mouse. Pregnant mice were screened by PCR using a tail DNA and southern blot analysis method.

### (Tumor growth and metastasis of mouse with human and mouse heterografts)

In order to study the growth of primary tumor, 1 ml of PBS solution containing ALVA-41 cells (5 x 10⁶ cells) was injected subcutaneously into the right flank of a male nude mouse. Separately, a solution of mouse melanoma B16 cells (1 x 10⁶ cells) was likely injected subcutaneously into the right flank of an inbred wild-type (WT) mouse, CatE^{-/-} mouse and CatE^{Tg} mouse. The size of the tumor was measured with a caliper, and the tumor volume was calibrated by ab²/2 (where a and b represent a maximum dimension of the central section and a minimum dimension of the central section, respectively). As the size of the heterograft of the ALVA-41 cell reached approximately 100 mm³ (approximately at day 12 after injection), animals were divided into two groups of each 5 animals. To the two animal groups, respectively, purified cathepsin E (200 µg per kg of body weight per day) and physiological saline were injected into the central portion of the tumor for 16 days. For a measurement for the tumor weight and an immunohistochemical study, cathepsin E (500 µg per kg per day) or a solvent (polyethylene glycol) was injected into the central portion of the tumor for 10 days when the size of the tumor reached approximately 400 mm³. For a study on metastasis, melanoma B16 cells (2 x 10⁵ cells in 50 µl of PBS solution) was injected into a tail vein of a Wt mouse, CatE^{-/-} mouse and CatE^{Tg}. The mice were then anesthetized at day 22 after injection and killed with a lethal amount of diethylether, followed by removing the lungs and counting the number of black metastasis colonies.

### (Extraction of mRNA and RT-PCR)

A total RNA was isolated with RNeasy Mini Kit in accordance with a manufacturer's manual. The yield and quality of RNA were assessed by absorption and gel electrophoresis with A260/A280. The cDNA synthesis was carried out with Ready-to-Go RT-PCR beads. Each sample (500 ng) was incubated in 50 µl of a reaction mixture of a first-strand primer, forward primer and reverse primer (each 20 pmol) and RNase/DNase-free water. RT was carried out by a heating program of 45 °C for 10 minutes and 95 °C for 5 minutes. The PCR reaction was carried out using the following primers: 5'-TCCACCACCCTGTTGCTGTA (forward primer) and 5'-ACCACAGTCCATGCCATCAC (reverse primer) for G3PDH as well as 5'-GTGCCCCTCAGAAGACATCA (forward primer) and 5'-GTATCCCAGACCCAGAATCC (reverse primer) for cathepsin E. The PCR products were 471 bp and 498 bp, respectively. The PCR cycle condition for G3PDH was such that a single cycle was made up by the first step at 95 °C for 30 seconds, the second step at 60 °C for 30 seconds, and the third step at 72 °C for 1 minute and 35 cycles were repeated continuously, followed by the last step in which the reaction was carried out at 72 °C for 5 minutes. All the samples were then decomposed on a 2.5% agarose gel containing ethidium bromide (0.5 µg/ml) and visualized by ultraviolet irradiation. In order to ensure that the detection was made within the linear range, PCR was carried at different cycles and repeated at least two times.

### (Statistical analysis)

Data are expressed as an average value ± SD and a comparison between the groups was made by a Student's test. Data on survival were analyzed by Mann-Whitney U-test and it was determined that a value of p<0.05 was statistically significant.

The following is a detailed description regarding the drawings affixed hereto.
Fig. 1 shows the effect of various cathepsins on the survival of human prostate cancer ALVA-41 cells. Fig. 1A shows the results of measurement by colorimetry for the counts of alive cells after the cells were treated with physiological saline and cathepsin B, D, E and L (each having a concentration of 10 µg/ml (on the left-hand side), 50 µg/ml (at the center) and 100 µg/ml (on the right-hand side) at 37°C and pH 7.4 for 20 hours. The data in the figures are each a relative value with respect to the value of the cells treated with physiological saline and are represented as an average value ± SD of four data obtained by four separate experiments. In the data, *p<0.001 represents a corresponding value with respect to the cells treated with physiological saline. Fig. 1B shows the result of microscopic observation about the cells treated with the various cathepsins (each having a concentration of 100 µg/ml) and physiological saline. In the figure, an indicating bar scale represents 50 µm. Fig. 1C shows an apoptosis pattern detected by treating the cells with physiological saline, cathepsin E (50 µg/ml) and cathepsin L (100 µg/ml) at 37 °C and pH 7.4 for 20 hours and carrying out the TUNNEL assay (on the upper panel) and fluorescent isothiooyanate-labeled annexin A staining (on the lower panel). In the figure, an indicating bar scale represents 50 µm.

Fig. 2 shows features of cathepsin E-induced apoptosis in tumor cells. Fig. 2A shows the result of measurement for the survival rate of ALVA-41 cells. In this experiment, the survival rate of the ALVA-41 cells was measured by incubating the cells with cathepsin E of a given concentration in the presence or absence of pepstatin A (100 µM) at 37 °C and pH 7.4 for 20 hours. The data is represented as a. percentage with respect to the value for non-treated cell group. The data were obtained as an average value ± SD from the data obtained by four separate experiments. In the value, *p<0.001 is a corresponding value for the cells treated with the cells non-treated with pepstatin A. Fig. 2B shows the result of measurement for the survival rate of HEK293 cells. In this experiment, the HEK293 cells were incubated together with a given concentration of natural cathepsin E, wild-type recombinant and the active site mutant (D98A/D283A) at 37 °C and pH 7.4 for 20 hours. The survival rate of the cells was measured and represented as a percentage with respect to the value of the non-treated cell group. The data represents an average value ± SD from the data obtained by four separate experiments. In the value, *p<0.001 is a corresponding value for the cells treated with the mutant. Fig. 2C shows a survival rate of cells treated with cathepsin E only. In this experiment, ALVA-41 cells were treated solely with cathepsin E of a given concentration in the same manner as for Fig. 2A and the culture supernatant was recovered, followed by centrifuging to remove the precipitate and transferring a portion (100 µM) of the supernatant to a freshly prepared culture of ALVA-41 cells. Thereafter, the cells were incubated at 37 °C and pH 7.4 for 20 hours in the absence (black round mark) or presence (white round mark) of pepstatin A (100 µM) and measured for the survival rate of the cells. The cell survival rate is represented as a percentage with respect to the value of the cells treated with the culture supernatant of the ALVA-41 cells treated with physiological saline. The data is represented as an average value ± SD from the data obtained by four separate experiments. In the experiment as shown in Fig. 2D, a culture supernatant of ALVA-41 cells treated with cathepsin E was fractioned by DEAE-Sephacel chromatography and each fraction was treated in the same manner as described above. The cathepsin E activity (on the upper panel) and the cytotoxic activity (on the lower panel) of each column fraction were determined using freshly prepared ALVA-41 cells and a cathepsin E-specific substrate. The cell survival rate of each fraction treated with the protein at a given final concentration is represented as a percentage with respect to the value of the cells treated with physiological saline and as an average value ± SD from the data obtained by four separate experiments. The cathepsin E activity is represented as a percentage of the total activity in the culture supernatant, and the data is represented as an average ± SD from the data obtained by three separate experiments.

Fig. 3 illustrates the identification of TRAIL as an inducing factor of cathepsin E-induced apoptosis in ALVA-41 cells. Fig. 3A shows the results of measurement for amounts of TNF-α, FasL and TRAIL in a culture supernatant of ALVA-41 cells. In this experiment, the ALVA-41 cells were treated with cathepsin E (100 µg/ml) or physiological saline at 37 °C and pH 7.4 for 20 hours and measured for the amounts of TNF-α, FasL and TRAIL in the culture supernatant by ELSA. The data is represented as a corresponding value with respect to an average value of the cells treated with physiological saline and as an average value ± SD from the data obtained by four separate experiments. Fig. 3B shows a result of the immunoblot analysis. In this experiment, the cells were treated with cathepsin E (lanes 2 and 4) or physiological saline (lanes 1 and 3) in the same manner as for Fig. 3A, and the immunoblot analysis for the cell extract and the culture supernatant was carried out using an antibody specific to each of TNF-α, FasL and TRAIL. Fig. 3C shows the result of the effect of suppression of the apoptosis-inducing activity by immunodepletion of TRAIL. In this experiment, TRAIL was removed by immunoprecipitation from a 0.1M NaCl fraction obtained by DEAE-Sephacel column chromatography of the culture supernatant ofthe cells treated with cathepsin E at a given final concentration using an anti-TRAIL antibody. As a control, the cells were treated in substantially the same manner as above using an antibody to a control mouse IgG. Apoptosis of the ALVA-41 cells was measured using the culture supernatant after immunoprecipitation. The data are represented as a percentage of a corresponding value for the survival rate of the cells treated with physiological saline and as an average value ± SD from the data obtained by three separate experiments. In the figure, *p<0.001 is a corresponding value with respect to the cells treated with the control IgG.

Fig. 4 illustrates sensitivity of various human prostate cancer cell lines to cathepsin E-induced apoptosis.
Fig. 4A shows the result of colorimetric measurements for the cell survival rate of various human prostate cancer cell lines and normal human prostate epithelial (PrE) cells, which were treated with cathepsin E or physiological saline. In this assay, the cells to be tested were incubated to a ∼80% confluent state and then together with cathepsin E (100 µg/ml) or physiological saline at 37 °C and pH 7.4 for 20 hours, followed by measurements for the cell survival rate by a colorimetric assay. The cell survival rate is represented as a percentage of a corresponding value to the cells treated with physiological saline, and the data is represented as an average value ± SD from the data obtained by four separate experiments. In this value, *p<0.001 represents a corresponding value to the cells treated with physiological saline. Fig. 4B shows the result of an immunoblot analysis. In this experiment, the cells were biotinylated with sulfo-NHS-SS-biotin as a cell-permeable, decomposable reagent and lyzed, and the resulting cell lysate was incubated with streptoavidin-agarose beads at 4 °C for 2 hours. After the beads were washed, the related proteins were subjected to an immunoblot analysis using an anti-TRAIL antibody or an antibody to a TRAIL membrane-related receptor. The cell lysate portion was also subjected to a direct immunoblot analysis using an anti-β-actin antibody. As an alternative method, a culture medium of each cell line was immunoblot-analyzed using an anti-OPG antibody. Fig. 4C shows the result of a concentration analysis with a densitometer. In this experiment, the culture supernatant treated with cathepsin E in the same manner as for Fig. 3A was subjected to immunoblot analysis using an anti-TRAIL antibody. Thereafter, the immunoblot was analyzed for its concentration in order to determine a quantity of a soluble TRAIL trimer. In the table, the unit of the ordinate is defined as a corresponding value to the TRAIL concentration per ml unit of PrE cells treated with cathepsin E. Data is represented as an average value ± SD from the data obtained by four separate experiments. In the figure, lane 1 shows PrE cells, lane 2 shows ALVA-41 cells, lane 3 shows ALVA-101 cells, lane 4 shows PPC-1 cells, lane 5 shows PC-3 cells, and lane 6 shows DU145 cells. In the value, *p<0.001 represents a statistically significant value for the PrE cells treated with cathepsin E.

Fig. 5 illustrates the effect of cathepsin E on the growth of tumors formed in nude mice transplanted with ALVA-41 cells.
Fig. 5A shows a volume of the tumor formed by subcutaneously injecting male nude mice with ALVA-41 cells. As the tumor volume reaches ∼ 100 cm³ after subcutaneous injection, cathepsin E (200 µg/kg/day) or physiological saline was subcutaneously injected into the central portion of the tumor for 16 days. Data are represented as an average value ± SD of the value obtained from five mice per each group, and *p<0.005, **p<0.01 and ***p<0.001 were statistically significant values corresponding to the mice treated with cathepsin E. Fig. 5B shows the result of measurements for the weight of tumor formed by injecting cathepsin E (400 µg/kg/day) or physiological saline for 10 days at the central portion of the tumor. Data arc represented as an average value ± SD of the value obtained from three mice per each group. Fig. 5C shows the result of analysis by TUNEL assay of the tumor of mice treated with cathepsin E or physiological saline in the same manner as for Fig. 5B. The TUNEL assay was carried out by reacting with diaminobenzidine and counter-staining tissue slices with 1% methyl green. Data represent the result obtained from five mice per group. In the figure, the lower image is an expanded version of the upper image. In the figure, a bar scale represents 50 µm.

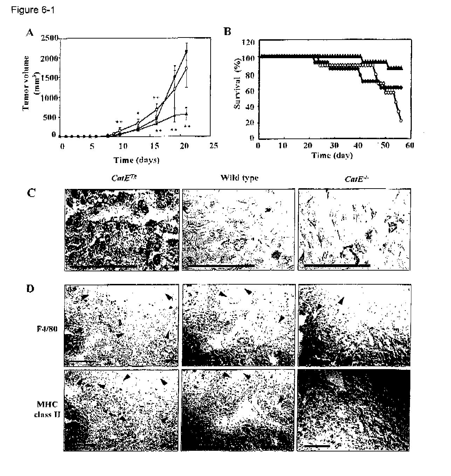

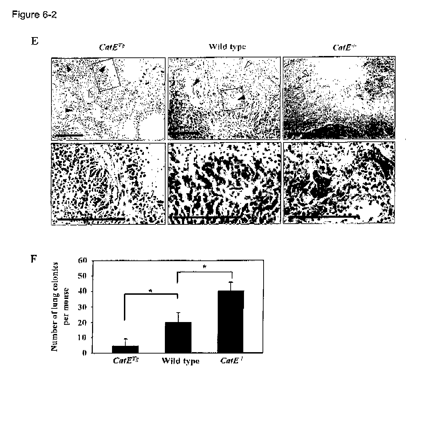

Figs. 6-1 and 6-2 illustrate a direct correlation of an expression level of endogenous cathepsin E with a decrease in the growth and metastasis of mouse B16 melanoma cells in the tumor.
Fig. 6A shows the result of measurements for a volume of the tumor formed by subcutaneous injection of each of CatE^{-/-} mice (n=11), Wt mice (n=10) and CatE^{Tg} mice (n=10) with inbred mouse B16 melanoma cells (1 x 10⁶ cells). The tumor volume was measured at a lapse of a predetermined time after injection and data was represented as an average value ± SD from each group value. In the value, *p<0.01 and **p<0.001 were statistically significant values for the corresponding mice. Fig. 6B shows the mortality rate of mice of each group after subcutaneous injection of mouse B16 melanoma cells. This figure indicates a statistically significant difference between the Wt mice and CatE^{-/-} mice or CatE^{Tg} mice. Fig. 6C shows the result of an analysis of TUNFL assay for the tumors removed from the mice of each group. The assay was carried out by reaction with diaminobenzidine and counter-staining of sliced tissues with 1% methyl green. The data is represented as the result at day 23 after transplantation into five mice of each group. In the figure, a bar scale represents 50 µm. Fig. 6D shows the result of immunohistochemical staining of the tumor removed from mice of each group in day 23 after transplantation with anti-F4/80 antigen antibody and anti-MHC class II molecule antibody. In the figure, the arrow mark indicates blood vessels and a bar scale represents 100 µm. Fig. 6E shows the result of paraformamide fixation and hematoxylin or eosin staining of the tumor removed at day 23 after transplantation with the cancer cells. In the figure, white arrow head mark and block arrow head mark indicate a blood vessel region and a necrosis region, respectively, in the tumor. The lower figure is an expanded version of the image (as indicated inside a square portion) of the upper figure. In the lower figure, a structure of angiogenesis is recognized in the central tumor portion for the CatE^{-/-} mouse. Fig. 6F is a graph showing the number of black nodules formed on the lung surface by intravenous injection of mouse B16 cells into mice of each group. In this experiment, the mice of each group were injected from tail veins with mouse B16 melanoma cells and killed in 22 days to count the number of black nodules on the lung surface. Data are represented as an average value ± SD of each group value. In the value, *p<0.01 and **p<0.001 represent each statistically significant values for the corresponding mice.

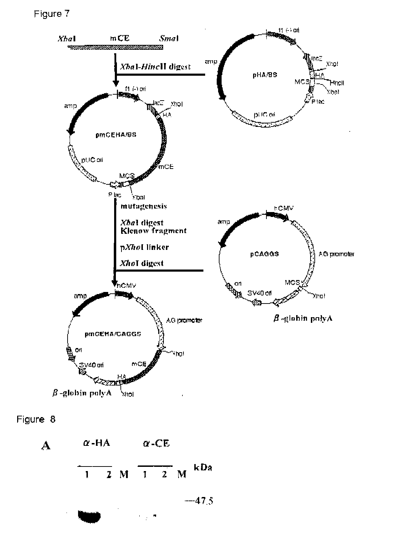

Fig. 7 is an illustration for the plasmid construction of mouse cathepsin E transgene. Cathepsin E labeled with HA was cloned on a target vector pCAGGS. The abbreviations in the figure mean as follows: amp for AG promoter; fl(-) ori for ampicillin-resistant gene; HA for haemagglutinin; hCMV for human cytomegalovirus immediate promoter; lacZ for β-galactosidase-α-fragment-β-globin promoter; mCE for rat cathepsin E gene; MCS for multiple cloing site; ori for E. coli plasmid pMB1 origin; P lac for lac promoter; pUC ori for pUC origin; and SV40 on for simian virus origin.

Fig. 8 shows data of immunoblot analysis for a cell extract of HEK193 cells with mouse cathepsin E overexpressed therein. In the analysis, antibodies to cathepsin E (CE) and hemagglutinin (HA) were used as a primary antibody. Lane 1 shows the cell with cathepsin E tranfected, and lane 2 shows a control cell.

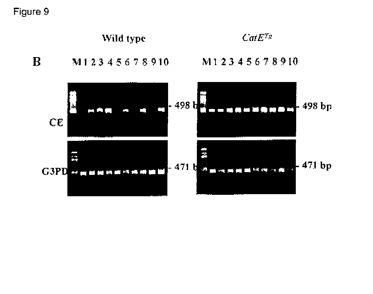

Fig. 9 shows amounts of expression of cathepsin E mRNA in various mouse organs of transgenic mouse (CatE^{Tg} mouse) with cathepsin E overexpressed and wild type (Wt) mouse as a control. In the figure, M means a 100-base pair marker, and G3PDH means glycyl aldehyde 3 phosphate dehydrogenase. Lanes 1 to 10, inclusive, refer to brain, heart, lungs, stomach, pancreas, spleen, small intestine, large intestine, liver and kidney, respectively.

### [Example 2]

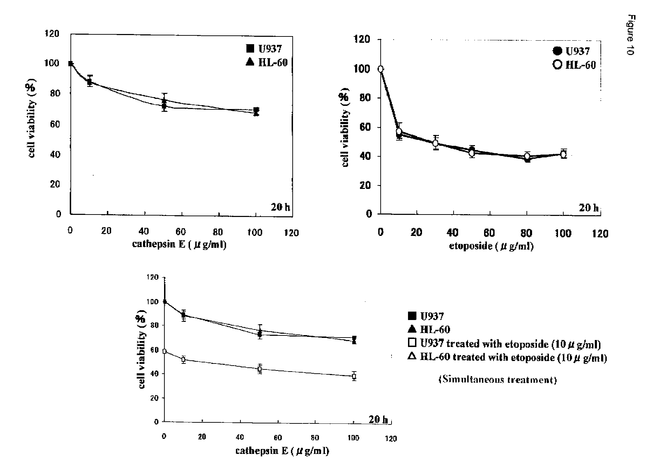

Fig. 10 shows the result of investigation on the influence of cathepsin E and etoposide upon the survival of human leukemia cells. The upper panel shows the result of a review on influences on concentrations of administration when leukemia U937 cells and HL-60 cells were administered with cathepsin E and etoposide. The lower panel shows the result of the effect of a simultaneous administration of etoposide (10 µg/ml) and cathepsin E of various concentrations to the respective cells compared to the sole administration of cathepsin E. These results indicate a great decrease in the survival rate of each cancer cells when cathepsin E and etoposide were administered simultaneously.

### [Example 3]

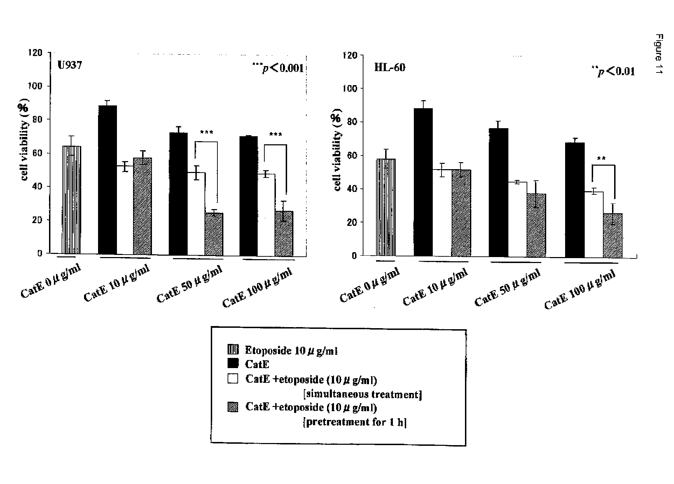

Fig. 11 shows the result of investigation on the influence of cathepsin E and etoposide on the survival of human leukemia cells, compared with the case of a simultaneous administration of cathepsin E and etoposide and the case of pretreatment with etoposide. As shown herein, it was found that apoptosis of the cancer cells was enhanced to a remarkable extent when cathepsin E of various different concentrations and etoposide were administered simultaneously and the cells were pretreated with etoposide, as compared with the case of a sole administration of etoposide (10 µg/ml) where etoposide induced apoptosis of human leukemia U937 cells only at a rate of approximately 40%.

### [Example 4]

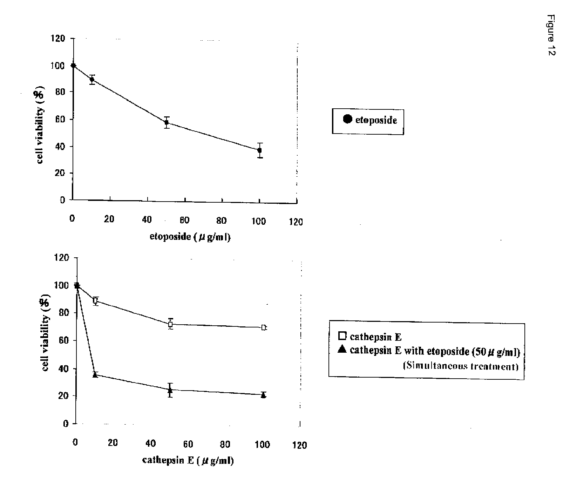

Fig. 12 shows the effect upon the survival of human leukemia U937 cells when cathepsin E and etoposide were administered in combination and when etoposide was administered solely. The upper panel shows the effect of a sole administration of etoposide and the lower panel shows the effects of a combination administration of etoposide (50 µg/ml) and cathepsin E of different concentrations. These results were found that the effect of etoposide was enhanced to an apparent extent by a combination of etoposide with cathepsin E.

### INDUSTRIAL APPLICABILITY

The anticancer cathepsin formulation according to the present invention is useful for the suppression of growth of cancer cells and prevention of cancer metastasis and can induce apoptosis of tumor cells to a remarkable extent. The anticancer cathepsin formulation of the present invention does not adversely affect normal cells, but exerts damages only upon cancer cells so that it may cause no side effects, which may be caused otherwise by conventional anticancer agents, or eliminate such side effects to a remarkable extent. Further, as the cathepsin formulation of the present invention can be expected to produce a therapy effect upon cancers to which conventional anticancer agents cannot demonstrate sensitivity, particularly when it is used in combination with such conventional anticancer agents. Therefore, the cathepsin formulation of the present invention is extremely useful particularly in the field of cancer therapy.
(Reference No. 1)
[Table 1]
1. Ashkenazi, A., and V.M. Dixit. 1998. Death receptors: signaling and modulation. Science 281: 1305-1308.
2. Wiley, S.R., K. Schooley, P.J. Smolak, W.S. Din, C.P. Huang, J.K. Nicholl, GR. Sutherland, T.D. Smith, C. Rauch, C,A. Smith, and R.G. Goodwin. 1995. Identification and characterization of a new member of the TNF family that induces apoptosis. Immunity 3: 673-682.
3. Pitti, R.M., S.A. Marsters, S. Ruppert, C.J. Donahue, A. Moore, and A. Ashkenazi. 1996. Induction of apoptosis by Apo-2 ligand, a new member of the tumor necrosis factor cytokine family. J Biol Chem 271: 12687-12690.
4. van Ravenswaay, C.H.H., P.M. Kluin, and G.J. Fleuren. 1992. Tumor infiltrating cells in human cancer. On the possible role of CD16+ macrophages in antitumor cytotoxicity. Lab Invest 67: 166-174.
5. Naama, H.A., V.E. Mack, G.P. Smyth, P.P. Stapleton, and J.M. Daly. 2001. Macrophage effector mechanisms in melanoma in an experimental study. Arch Surg 136: 804-809,
6. Varney, M,L., K.J. Olsen, R.L. Mosley, C.D. Bucana, J.E. Talmadge, and R.K. Singh. 2002 Monocyte/macrophage recruitment, activation and differentiation modulate interleukin-8 production: a paracrine role of tumor-associated macrophages in tumor angiogenesis. In Vivo 16: 471-477.
7. Nomura, T" and N. Katsunuma. 2005. Involvement of cathepsins in the invasion, metastasis and proliferation of cancer. J Med Invest 52: 1-9.
8. Overall, C.M., and O. Kleifeld. 2006. Tumour microenvironment-opinion: validating matrix metalloproteinases as drug targets and anti-targets for cancer therapy. Nat Rev Cancer 6: 227-239.
      (Reference No. 2)
   [Table 2]
9. Sastradipura, D,F" H. Nakanishi, T, Tsukuba, K. Nishishita, H. Sakai, Y. Kato, T. Gotow, Y. Uchiyama, and K. Yamamoto. 1998. Identification of cellular compartments involved in processing of cathepsin E in primary cultures of rat microglia. J Neurochem 70: 2045-2056.
10. Nishioku, T., K. Hashimoto, K. Yamashita, S.Y. Liou, Y. Kagamiishi, H. Maegawa, N. Katsube, C. Peters, K. von Figura, P. Saftig, P., N. Katsunuma, K. Yamamoto, and H. Nakanishi. 2002. Involvement of cathepsin E in exogenous antigen processing in primary cultured murine microglia. J Biol Chem 277: 4816-4822.
11. Sakai, H., T. Saku, Y. Kato, and K. Yamamoto. 1989. Quantitation and immunohistochemical localization of cathepsin E and D in rat tissues and blood cells. Biochim Biophys Acta 991: 367-375.
12. Yanagawa, M., T. Tsukuba, K. Okamoto, R. Takii, Y. Terada, T. Kadowaki, and K. Yamamoto. 2006. Up-regulation, Enhanced Maturation, and Secretion of Cathepsin E in Mouse Macrophages Treated with Interferon-γ or Lipopolysaccharide, J Oral Biosci 48: 218-225.
13. Yamamoto, K. 1999. Cathepsin E and cathepsin D. In Proteases: New Perspectives. V. Turk. editor: Birkhäuser Verlag, Basel; 59-71.
14. Bennett, K., T. Levine, J.S. Ellis, R.J. Peanasky, I.M. Samioff, J. Kay, and B.M. Chain. 1992. Antigen processing for presentation by class II major histocompatibility complex requires cleavage by cathepsin E. Eur J Immunol 22: 1519-1524.
15. Sealy, L" F. Mota, N. Rayment, P. Tatnell, J. Kay, and B. Chain. 1996. Regulation of cathepsin E expression during human B cell differentiation in vitro. Eur. J. Immunol. 26: 1838-1843.
16. Chain, B.M., P. Free, P. Medd, C. Swetman, A.B. Tabor, and N. Terrazzini. 2005. The (Reference No. 3) [Table 3] expression and function of cathepsin E in dendritic cells. J Immunol 174: 1791-1800.
17. Tsukuba, T., K. Okamoto, Y. Okamoto, M. Yanagawa, K. Kohmura, Y. Yasuda, H. Uchi, T. Nakahara, M. Furue, K. Nakayama, T. Kadowaki, K. Yamamoto, and K.I. Nakayama. 2003. Association of cathepsin E deficiency with development of atopic dermatitis. J. Biochem. 134: 893-902.
18. Tsukuba, T., S. Yamamoto, M. Yanagawa, K. Okamoto, Y. Okamoto, K.I. Nakayama, T. Kadowaki, and K. Yamamoto. 2006. Cathepsin E-deficient mice show increased susceptibility to bacterial infection associated with the decreased expression of multiple cell surface Toll-like receptors. J Biochem 140: 57-66.
19.Yanagawa, M., T. Tsukuba, T. Nishioku, T., Y. Okamoto, K. Okamoto, R. Takii, Y. Terada, K.I. Nakayama, T. Kadowaki, and K. Yamamoto. 2007. Cathepsin E deficiency induces a novel form of lysosomal storage disorder showing the accumulation of lysosomal membrane sialoglycoproteins and the elevation of lysosomal pH in macrophages. J Biol Chem 282: 1851-1862.
20. Ullmann, R., P. Morbini, I. Halbwedl, M. Bongiovanni, M. Gogg-Kammerer, M. Papotti, S. Gabor, H, Renner, and H,H. Popper. 2004. Protein expression profiles in adenocarcinomas and squamous cell carcinomas of the lung generated using tissue microarrays. J Pathol 203: 798-807.
21. Busquets, L., H. Guillen, M.E. DeFord, M.A. Suckow, R.M. Navari, F.J. Castellino, and M. Prorok. 2006. Cathepsin E is a specific marker of dysplasia in APC mouse intestine. Tumor Biol 27: 36-42.
22. Blaveri, E., J.P Simko, J.E. Korkola, J.L. Brewer, F/ Bachner, K. Mehta, S. Devries, T. Koppie, S. Pejavar, P. Carroll, and F.M. Waldman. 2005. Bladder cancer outcome and [0089] (Reference No. 4) [Table 4] subtype classification by gene expression. Clin Cancer Res 11: 4044-4055.
23. Wild, P.J., A, Herr, C. Wissmann, R. Stoehr, A. Rosenthal, D. Zaak, R. Simon, R. Knuechel, C. Pilarsky, and A. Hartmann. A. 2005. Gene expression profiling of progressive papillary noninvasive carcinomas of the urinary bladder. Clin Cancer Res 11: 4415-4429.
24. Kirschke, H" R. Eerola, V.K. Hopsu-Havu, D. Brömme, and E. Vuorio. 2000. Antisense RNA inhibition of cathepsin L expression reduces tumorigenicity of malignant cells. Eur J Cancer. 36: 787-795.
25. Krueger, S., U. Kellner, F. Buehling, and A. Roessner. 2991, Cathepsin L antisense oligonucleotides in a human osteosarcoma cell line: effects on the invasive phenotype. Cancer Gene Ther. 8: 522-528.
26. Levicar, N" R.A. Dewey, E. Daley, T.E. Bates, D. Davies, J. Kos, G.J. Pilkington, and T.T. Lah. 2003. Selective suppression of cathepsin L by antisense cDNA impairs human brain tumor cell invasion in vitro and promotes apoptosis. Cancer Gene Ther. 2003; 10: 141-151.
27. Yamamoto, K., N. Katsuda, and K. Kato. 1978. Affinity purification and properties of cathepsin E-like acid proteinase from rat spleen. Eur J Biochem 92: 499-508.
28. Yamamoto, K" and V.T. Marchesi. 1984. Purification and characterization of acid proteinase from human erythrocyte membranes. Biochim Biophys Acta. 790: 208-218.
29. Kimberley, F.C., and G.R. Screaton. 2004. Following a TRAIL: update on a ligand and its five receptors. Cell Res. 14: 359-372.
30. Seol, D., and T.R. Billiar. 2000. Cysteine 230 modulates tumor necrosis factor-related apoptosis-inducing ligand activity. Cancer Res 60: 3152-3154.
31. Bodmer. J., P. Meier, J. Tschopp, and P. Schneider. 2000. Cysteine 230 is essential for the structure and activity of the cytotoxic ligand TRAIL. J Biol Chem 275: 20632-20637.
32. Hymowitz, S.G., M.P. O'Connell, M.H. Ultsch, A, Hurst, K. Totpal, A. Aschkenazi, A.M. Vos, and R.F. Kelly. 1999. Triggering cell death: the crystal structure of Apo2L/TRAIL in a complex with death receptor 5. Moll Cell 4: 563-571.
      (Reference No. 5)
   [Table 5]
33. Griffith, T.S., and D.H. Lynch. 1998. TRAIL: a molecule with multiple receptors and control mechanisms. Curr Opin Immunol 10: 559-563.
34. Emery, J.G., P. MeDonnell, M.B. Burke, K.C. Deen, S. Lyn, C. Silverman, E. Dul, E. R. Appelbaum, C. Eichman, R. DiPrinzio, R.A. Dodds, I.E. James, M. Rosenberg, J.C. Lee, and P.R. Young. 1998. Osteoprotegerin is a receptor for the cytotoxic ligand TRAIL. J Biol Chem. 273:14363-14367.
35. Holen, I., P.I. Croucher, F.C. Hamdy, and C.L. Eaton. 2002. Osteoprotegerin (OPG) is a survival factor for human prostate cancer cells. Cancer Res. 62: 1619-1623.
36. Andreesen, R., B. Hennemann, and S.W. Krause. 1998. Adoptive immunotherapy of cancer using monocyte-derived macrophages: rationale, current status, and perspectives. J Leukoc Biol 64: 419-426.
37. Griffith, T.S., S.R. Wiley, M.Z. Kubin, M.L. Sedger, C.R. Maliszewski, and N.A. Fanger. 1999. Monocyte-mediated tumoricidal activity via the tumor necrosis factor-related cytokine, TRAIL. J Exp Med 189: 1343-1353.
38. Kayagaki, N., N. Yamaguchi, M. Nakayama, K. takeda, H. Akiba, H. Tsutsui, H. Okayama, K. Nakanishi, K. Okayama, and H. Yagita. 1999. Type I interferons (IFNs) regulate tumor necrosis factor-related apoptosis-inducing ligand (TRAIL) expression on human T cells: A novel mechanism for the antitumor effects of type I IFNs. J Exp Med 189: 1451-1460.
39. Fanger, N.A., C.R. Maliszewski, K. Schooley, and T.S. Grifith. 1999. Human dendritic cells mediate cellular apoptosis via tumor necrosis factor-related apoptosis-inducing ligand (TRAIL). J Exp Med. 190: 1155-64.
      (Reference No. 6)
   [Table 6]
40. Nishishita, K., H. Sakai, E. Sakai, Y. Kato, and K. Yamamoto. 1996. Age-related and dexamethasone-induced changes in cathepsins E and D in rat thymic and splenic cells. Arch Biochem Biophys. 333: 349-358.
41. Takeda, K, M.J. Smyth, E. Cretney, Y. Hayakawa, N. Yamaguchi, H. Yagita, and K. Yamamoto. 2001. Involvement of tumor necrosis factor-related apoptosis-inducing ligand in NK cell-mediated and IFN-gamma-dependent suppression of subcutaneous tumor growth. Cellular Immunol 214: 194-200.
42. Weissler, J.C., M.F. Lipscomb, V.M. Lem, and G.B. Toews. 1986. Tumor killing by human alveolar macrophages and blood monocytes. Decreased cytotoxicity of human alvcolar macrophages. Am Rev Respir Dis. 134; 532-537.
43. Yanagawa, H., E. Takeuchi, Y. Suzuki, Y. Ohmoto, H. Bando, S. Sone. 1999. Presence and potent immunosuppressive role of interlcukin-10 in malignant pleural effusion due to lung cancer. Cancer Lett 136: 27-32.
44. Ding, A.H., C.F. Nathan, and D.J. Stuehr. 1988. Release of reactive nitrogen intermediates and reactive oxygen intermediates from mouse peritoneal macrophages. Comparison of activating cytokines and evidence for independent production. J Immunol 141: 2407-2412.
45. Steplewski, Z., M.D. Lubeck, and H. Koprowski. 1983. Human macrophages armed with murine immunoglobulin G2a antibodies to tumors destroy human cancer cells. Science 221:865-867,
46. te Veldc, A.A., and C.C. Figdor. 1992. Monocyte mediated cytotoxic activity against melanoma. Melanoma Res 1: 303-309.
47. Black, R.A., C.T. Rauch, C.J. Kozlosky, J.J. Perschon, J.L. Slack, M.F. Wolfson, B.J. Castner, K.L. Stocking, P. Reddy, S. Srinvasan, N. Nelson, N. Boiani, K.A. Schooley, M.
      (Reference No. 7)
   [Table 7]
   Gerhart, R. Davis, J.N. Fitzner, R.S. Johnson, R.J. Paxton, C.J. March, and D.P. Cerretti. 1997. A metalloproteinase disintegrin that releases tumour-necrosis factor-alpha from cells. Nature 385: 729-733.
48. Moss, M.L., S.L. Jin, M.E. Milla, D.M. Bickett, W. Burkhart, H.L. Carter, W.J. Chen, W.C. Clay, J.R. Didsbury, D. Hassler, C.R. Hoffman, T.A. Kost, M.H. Lambert, M.A. Leesnitzer, P. McCauley, G. McGeehan, J. Mitchell, M. Moyer, G. Pahel, W. Rocque, L.K. Overton, F. Schoenen, T. Seaton, J.L. Su, J.D. Becherer, et al. 1997. Cloning of a disintegrin metalloproteinase that processes precursor tumour-necrosis factor-alpha. Nature; 385: 733-736.
49. Strand, S., P. Vollmer, L. van den Abeelen, D. Gottfried, V. Alla, H. Heid, J. Kuball, M. Theobald, P.R. Galle, and D. Strand. 2004. Cleavage of CD95 by matrix metalloproteinase-7 induces apoptosis resistance in tumour cells. Oncogene 23: 3732-3736.
50. Vargo-Gogola, T., H.C. Crawford, B. Fingleton, and L.M. Matrisian. 2002. Identification of novel matrix metalloproteinase-7 (matrilysin) cleavage sites in murine and human Fas ligand. Arch Biochem Biophys. 408: 155-161.
51. Mitsiades, N., W.H. Yu, V. Poulaki, M. Tsokos, and I. Stamenkovic. 2001. Matrix metalloproteinase-7-mediated cleavage of Fas ligand protects tumor cells from chemotherapeutic drug cytotoxicity, Cancer Res. 61: 577-581.
52. Mariani, S.M., and P.H. 1998. Crammer. Differential regulation of TRAIL and CD95 ligand in transformed cells of the T and B lymphocyte lineage. Eur J Immunol. 28: 973-982.
53. Kayagaki, N., N. Yamaguchi, M. Nakayama, H. Ito, K. Okumura, and H. Yagita. 1999. Type I interferons (IFNs) regulate tumor necrosis factor-related apoptosis-inducing ligand (TRAIL) expression on human T cells: A novel mechanism for the antitumor effects of type I IFNs. J [0093] (Reference No. 8) [Table 8] Exp Mad 189: 1451-1460.
54. Mariani, S.M., and P.H. Krammer. 1998. Surface expression of TRAIL/Apo-2 ligand in activated mouse T and B cells. Eur J Immunol 28: 1492-1498.
55. Kashii, Y,, R. Giorda, R.B. Herberman, T.L. Whiteside, and N.L. Vujanovic. 1999. Constitutive expression and role of the TNF family ligands in apoptotic killing of tumor cells by human NK cells. J Immunol 163: 5358-5366.
56. Leibovici, J., and S. Hoenig. 1985. Lysis and growth stimulation of a murine melanoma determined by density of macrophage populations. Anticancer Res. 5: 545-551.
57. Balkwill, F., K.A. Charles, and A. Mantovani. 2005. Smoldering and polarized inflammation in the initiation and promotion of malignant disease. Cancer Cell. 7: 211-217.
58. Hussein, M.R., 2006. Tumour-associated macrophages and melanoma tumorigenesis : integrating the complexity. Int J Exp Pathol 87: 163-176.
59. Condeelis J, and Pollard JW. Macrophages: obligate partners for tumor cell migration, invasion, and metastasis. Cell 2006; 124: 263-6.
60. Yamamoto K, Takeda M, Yamamoto H, Tatsumi M, Kato Y. Human erythrocyte membrane acid proteinase (EMAP): sidedness and relation to cathepsin D. J Biochem. 1985; 97: 821-30.
61. Bando, Y., E. Kominami, and N. Katunuma. 1986. Purification and tissue distribution of rat cathepsin L. J Biochem. 100:35-42.
62. Yamamoto, K., O. Kamata, M. Takeda, and Y. Kato. 1983. Characterization of rat spleen cathepsin B and different effects of anti-inflammatory drugs on its activity, Jpn J Oral Biol 25: 834-838.
63. Liu, J" T. Tsukuba, K. Okamoto, M. Onishi, and K. Yamamoto. 2002. Mutational analysis of [0094] SEQUENCE LISTING
   ...PCT.txt

## Claims

1. An anticancer cathepsin formulation **characterized in that** cathepsin E and/or an active fragment comprising or containing its active site as an effective ingredient and/is contained as an effective ingredient.

2. The anticancer cathepsin formulation as claimed in claim 1, wherein said active side is a region containing aspartic acid at positions 98 and 283 of an amino acid sequence as represented as SEQ ID #1 of the Sequence Listing.

3. The anticancer cathepsin formulation as claimed in claim 1 or 2, wherein an anticancer agent is further contained as an effective ingredient.

4. The anticancer cathepsins formulation as claimed in claim 3, wherein said anticancer agent is an agent effective for treating cancer or tumor.

5. The anticancer cathepsin formulation as claimed in claim 3 or 4, wherein said anticancer agent is a platinum-complex compound, topoisomerase inhibitor, antibiotic, anti-mitotic alkaloid or difluoronucleoside.

6. The anticancer cathepsin formulation as claimed in any one of claims 3 to 5, wherein said anticancer agent is applied to cancer or tumor selected from cancer of esophagus, throat, thyroid, stomach, liver, lungs (including vesicles), breast, pancreas, gallbladder, kidney, large intestine, rectum, colon, bladder, prostate gland, ovary, skin (including squamous cell carcinoma), etc.; hematopoietic leukemias of the lymphatic lineage, e.g., lymphocytic leukemia, lymphoblastic leukemia, cell leukemia, Hodgkin's lymphoma, Non-Hodgkin's lymphoma, etc.; hematopoietic leukemias of the myelogenous lineage, e.g., myelogenous leukemia, myelodysplastic syndromes, promyelocytic leukemia, etc.; sarcomas of the central and peripheral nervous systems, e.g., astrocytoma, neuroblastoma, glioma, schwannoma, etc., sarcomas of the mesenchymal system, e.g., fibrosarcoma, rhabdomyosarcoma, osteosarcoma, etc., other cancers, e.g., melanomas, xeroderma pigmentosum, keratinizing epithelioma, seminoma, thyroid folliculoma, or tertoma..

7. A method for enhancing sensitivity of an anticancer agent **characterized in that** the anticancer cathepsin formulation as claimed in any one of claims 1 to 6 comprising, as an effective ingredient, cathepsin E and/or an active fragment composed of or containing an active site thereof is combined with an anticancer agent.

8. The method for enhancing sensitivity of the anticancer agent as claimed in claim 7, wherein said active site is a region containing aspartic acid at positions 98 and 283 of amino acid sequence as represented by SEQ ID #1 of Sequence Listing.

9. The method for enhancing sensitivity of the anticancer agent as claimed in claim 7 or 8, wherein said anticancer agent is a cytotoxic anticancer agent selected from alkylating agent, an antimetabolite, an antibiotic and a microtubule-disrupting agent or a molecular target therapy agent.

10. The method for enhancing sensitivity of the anticancer agent as claimed in any one of claims 7 to 9, wherein said anticancer agent is applied to cancer or tumor selected from cancer of esophagus, throat, thyroid, stomach, liver, lungs (including vesicles), breast, pancreas, gallbladder, kidney, large intestine, rectum, colon, bladder, prostate gland, ovary, skin (including squamous cell carcinoma), etc.; hematopoietic leukemias of the lymphatic lineage, e.g., lymphocytic leukemia, lymphoblastic leukemia, cell leukemia, Hodgkin's lymphoma, Non-Hodgkin's lymphoma, etc.; hematopoietic leukemias of the myelogenous lineage, e.g., myelogenous leukemia, myelodysplastic syndromes, promyelocytic leukemia, etc.; sarcomas of the central and peripheral nervous systems, e.g., astrocytoma, neuroblastoma, glioma, schwannoma, etc., sarcomas of the mesenchymal system, e.g., fibrosarcoma, rhabdomyosarcoma, osteosarcoma, etc., other cancers, e.g., melanomas, xeroderma pigmentosum, keratinizing epithelioma, seminoma, thyroid folliculoma, or tertoma.

11. A method for TRAIL expression **characterized in that** TRAIL is expressed on the surface of a cancer cell by bringing cathepsin E and/or an active fragment comprising or containing an active site thereof into contact with the cancer cell.

12. The method for TRAIL expression as claimed in claim 11, wherein said active site is a region containing aspartic acid at positions 98 and 283 of amino acid sequence as represented by SEQ ID #1 of Sequence Listing.

13. A method for destroying cancer cells **characterized in that** cathepsin E and/or an active fragment comprising or containing an active site thereof is brought into contact with cancer cells to express TRAIL and to substantially destroy the cancer cells only without adversely affecting normal cells.

14. The method for destroying cancer cells as claimed in claim 13, wherein said active site is a region containing aspartic acid at positions 98 and 283 of amino acid sequence as represented by SEQ ID #1 of Sequence Listing.

15. A method of cancer therapy **characterized in** treating cancer with an anticancer cathepsin formulation as claimed in any one of claims 1 to 6.

16. A method of cancer therapy **characterized in** treating cancer by using a method for enhancing sensitivity of an anticancer agent as claimed in any one of claims 7 to 10.

17. The method for cancer therapy as claimed in claim 15 or 16, wherein said anticancer agent is a cytotoxic anticancer agent selected from an antimetabolite, an alkylating agent, an antibiotic and a microtubule-disrupting agent or a molecular target therapy agent.

18. The method for cancer therapy as claimed in any one of claims 15 to 17, wherein said anticancer agent is applied to cancer or tumor selected from cancer of esophagus, throat, thyroid, stomach, liver, lungs (including vesicles), breast, pancreas, gallbladder, kidney, large intestine, rectum, colon, bladder, prostate gland, ovary, skin (including squamous cell carcinoma), etc.; hematopoietic leukemias ofthe lymphatic lineage, e.g., lymphocytic leukemia, lymphoblastic leukemia, cell leukemia, Hodgkin's lymphoma, Non-Hodgkin's lymphoma, etc.; hematopoietic leukemias of the myelogenous lineage, e.g., myelogenous leukemia, myelodysplastic syndromes, promyelocytic leukemia, etc.; sarcomas of the central and peripheral nervous systems, e.g., astrocytoma, neuroblastoma, glioma, schwannoma, etc., sarcomas of the mesenchymal system, e.g., fibrosarcoma, rhabdomyosarcoma, osteosarcoma, etc., other cancers, e.g., melanomas, xeroderma pigmentosum, keratinizing epithelioma, seminoma, thyroid folliculoma, or tertoma.

19. A TNF homotrimer **characterized in that** said TNF homotrimer is formed with a member or members of a tumor necrosis factor (TNF) family selected from TNF-α.FasL (CD95Lor Apo1L) and TRAIL (or Apo1L).


SEQUENCE LISTING

<110>  Kyushu University

<120>  Anticancer Cathepsin Formulations

<130>

<160>  4

<170>  PatentIn version 3.1

<210>  1
<211>  400
<212>  PRT
<213>  rat

<400>  1

Met Lys Pro Leu Phe Val Leu Leu Leu Leu Leu Leu Leu Leu Asp Leu
                5                   10                  15

Ala Gln Ala Gln Gly Val Leu His Arg Val Pro Leu Xaa Arg Arg His
            20                  25                  30

Gln Ser Leu Arg Lys Lys Leu Arg Ala Gln Gly Gln Leu Ser Asp Phe
        35                  40                  45

Trp Arg Ser His Asn Leu Asp Met Ile Glu Phe Ser Glu Ser Cys Asn
    50                  55                  60

Val Asp Lys Gly Ile Asn Glu Pro Leu Ile Asn Tyr Leu Asp Met Glu
65                  70                  75                  80

Tyr Phe Gly Thr Val Ser Ile Gly Ser Pro Ser Gln Asn Phe Thr Val
                85                  90                  95

Ile Phe Asp Thr Gly Ser Ser Asn Leu Trp Val Pro Ser Val Tyr Cys
            100                 105                 110

Thr Ser Pro Ala Cys Lys Ala His Pro Val Phe His Pro Ser Gln Ser
        115                 120                 125

Ser Thr Tyr Met Glu Val Gly Asn His Phe Ser Ile Gln Tyr Gly Thr
    130                 135                 140

Gly Ser Leu Thr Gly Ile Ile Gly Ala Asp Gln Val Ser Val Glu Gly
145                 150                 155                 160

Leu Thr Val Glu Gly Gln Gln Phe Gly Glu Ser Val Lys Glu Pro Gly
                165                 170                 175

Gln Thr Phe Val Asn Ala Glu Phe Asp Gly Ile Leu Gly Leu Gly Tyr
            180                 185                 190

Pro Ser Leu Ala Val Gly Gly Val Thr Pro Val Phe Asp Asn Met Met
        195                 200                 205

Ala Gln Asn Leu Val Ala Leu Pro Met Phe Ser Val Tyr Leu Ser Ser
    210                 215                 220

Asp Pro Gln Gly Gly Ser Gly Ser Glu Leu Thr Phe Gly Gly Tyr Asp
225                 230                 235                 240

Pro Ser His Phe Ser Gly Ser Leu Asn Trp Ile Pro Val Thr Lys Gln
                245                 250                 255

Gly Tyr Trp Gln Ile Ala Leu Asp Gly Ile Gln Val Gly Asp Thr Val
        260                 265                 270

Met Phe Cys Ser Glu Gly Cys Gln Ala Ile Val Asp Thr Gly Thr Ser
    275                 280                 285

Leu Ile Thr Gly Pro Pro Lys Lys Ile Lys Gln Leu Gln Glu Ala Ile
    290                 295                 300

Gly Ala Thr Pro Met Asp Xaa Gly Glu Tyr Ala Val Asp Cys Ala Thr
305                 310                 315                 320

Leu Asn Met Met Pro Asn Val Thr Phe Leu Ile Asn Gly Val Ser Tyr
                325                 330                 335

Thr Leu Ser Pro Thr Ala Tyr Ile Leu Pro Asp Leu Val Asp Gly Met
            340                 345                 350

Gln Phe Cys Gly Ser Gly Phe Gln Gly Leu Asp Ile Gln Pro Pro Ala
        355                 360                 365

Gly Pro Leu Trp Ile Leu Gly Asp Val Phe Ile Arg Lys Phe Tyr Ser
    370                 375                 380

Val Phe Asp Arg Gly Asn Asn Gln Val Gly Leu Ala Pro Ala Val Pro
385                 390                 395                 400

<210>  2
<211>  400
<212>  PRT
<213>  Homo sapiens

<400>  2

Met Lys Thr Leu Xaa Xaa Leu Leu Leu Leu Leu Val Leu Leu Glu Leu
                5                   10                  15

Gly Glu Ala Gln Gly Ser Leu His Arg Val Pro Leu Xaa Arg Arg His
            20                  25                  30

Pro Ser Leu Lys Lys Lys Leu Arg Ala Arg Ser Gln Leu Ser Glu Phe
        35                  40                  45

Trp Lys Ser His Asn Leu Asp Met Ile Gln Phe Thr Glu Ser Cys Ser
    50                  55                  60

Met Asp Gln Ser Ala Lys Glu Pro Leu Ile Asn Tyr Leu Asp Met Glu
65                  70                  75                  80

Tyr Phe Gly Thr Ile Ser Ile Gly Ser Pro Pro Gln Asn Phe Thr Val
                85                  90                  95

Ile Phe Asp Thr Gly Ser Ser Asn Leu Trp Val Pro Ser Val Tyr Cys
            100                 105                 110

Thr Ser Pro Ala Cys Lys Thr His Ser Arg Phe Gln Pro Ser Gln Ser
        115                 120                 125

Ser Thr Tyr Ser Gln Pro Gly Gln Ser Phe Ser Ile Gln Tyr Gly Thr
    130                 135                 140

Gly Ser Leu Ser Gly Ile Ile Gly Ala Asp Gln Val Ser Val Glu Gly
145                 150                 155                 160

Leu Thr Val Val Gly Gln Gln Phe Gly Glu Ser Val Thr Glu Pro Gly
                165                 170                 175

Gln Thr Phe Val Asp Ala Glu Phe Asp Gly Ile Leu Gly Leu Gly Tyr

180                    185                    190

Pro Ser Leu Ala Val Gly Gly Val Thr Pro Val Phe Asp Asn Met Met
        195                 200                 205

Ala Gln Asn Leu Val Asp Leu Pro Met Phe Ser Val Tyr Met Ser Ser
    210                 215                 220

Asn Pro Glu Gly Gly Ala Gly Ser Glu Leu Ile Phe Gly Gly Tyr Asp
225                 230                 235                 240

His Ser His Phe Ser Gly Ser Leu Asn Trp Val Pro Val Thr Lys Gln
                245                 250                 255

Ala Tyr Trp Gln Ile Ala Leu Asp Asn Ile Gln Val Gly Gly Thr Val
            260                 265                 270

Met Phe Cys Ser Glu Gly Cys Gln Ala Ile Val Asp Thr Gly Thr Ser
        275                 280                 285

Leu Ile Thr Gly Pro Ser Asp Lys Ile Lys Gln Leu Gln Asn Ala Ile
    290                 295                 300

Gly Ala Ala Pro Val Asp Xaa Gly Glu Tyr Ala Val Glu Cys Ala Asn
305                 310                 315                 320

Leu Asn Val Met Pro Asp Val Thr Phe Thr Ile Asn Gly Val Pro Tyr
                325                 330                 335

Thr Leu Ser Pro Thr Ala Tyr Thr Leu Leu Asp Phe Val Asp Gly Met
            340                 345                 350

Gln Phe Cys Ser Ser Gly Phe Gln Gly Leu Asp Ile His Pro Pro Ala
        355                 360                 365

Gly Pro Leu Trp Ile Leu Gly Asp Val Phe Ile Arg Gln Phe Tyr Ser
    370                 375                 380

Val Phe Asp Arg Gly Asn Asn Arg Val Gly Leu Ala Pro Ala Val Pro
385                 390                 395                 400

<210>  3
<211>  400
<212>  PRT
<213>  Guinea pig

<400>  3

Met Lys Thr Xaa Phe Xaa Leu Leu Leu Leu Leu Val Leu Leu Glu Leu
                5                   10                  15

Gly Gln Ala Pro Gly Ala Leu His Arg Val Pro Leu Ser Arg Arg Glu
            20                  25                  30

Xaa Ser Leu Arg Lys Lys Leu Arg Ala Gln Gly Gln Leu Thr Glu Leu
        35                  40                  45

Trp Lys Ser Gln Asn Leu Asn Met Asp Gln Xaa Xaa Xaa Xaa Cys Ser
    50                  55                  60

Thr Ile Gln Ser Ala Asn Glu Pro Leu Ile Asn Tyr Leu Asp Met Glu
65                  70                  75                  80

Tyr Phe Gly Thr Ile Ser Ile Gly Ser Pro Pro Gln Asn Phe Thr Val
                85                  90                  95

Ile Phe Asp Thr Gly Ser Ser Asn Leu Trp Val Pro Ser Val Tyr Cys
            100                 105                 110

Thr Ser Pro Ala Cys Gln Thr His Pro Val Phe His Pro Ser Leu Ser
        115                 120                 125

Ser Thr Tyr Arg Glu Val Gly Asn Ser Phe Ser Ile Gln Tyr Gly Thr
    130                 135                 140

Gly Ser Leu Thr Gly Ile Ile Gly Ala Asp Gln Val Ser Val Glu Gly
145                 150                 155                 160

Leu Thr Val Val Gly Gln Gln Phe Gly Glu Ser Val Gln Glu Pro Gly
                165                 170                 175

Lys Thr Phe Val His Ala Glu Phe Asp Gly Ile Leu Gly Leu Gly Tyr
            180                 185                 190

Pro Ser Leu Ala Ala Gly Gly Val Thr Pro Val Phe Asp Asn Met Met
        195                 200                 205

Ala Gln Asn Leu Val Ala Leu Pro Met Phe Ser Val Tyr Met Ser Ser
    210                 215                 220

Asn Pro Xaa Gly Gly Ser Gly Ser Glu Leu Thr Phe Gly Gly Tyr Asp
225                 230                 235                 240

Pro Ser His Phe Ser Gly Ser Leu Asn Trp Val Pro Val Thr Lys Gln
                245                 250                 255

Ala Tyr Trp Gln Ile Ala Leu Asp Gly Ile Gln Val Gly Asp Ser Val
            260                 265                 270

Met Phe Cys Ser Glu Gly Cys Gln Ala Ile Val Asp Thr Gly Thr Ser
        275                 280                 285

Leu Ile Thr Gly Pro Pro Gly Lys Ile Lys Gln Leu Gln Glu Ala Leu
    290                 295                 300

Gly Ala Thr Tyr Val Asp Glu Gly Xaa Tyr Ser Val Gln Cys Ala Asn
305                 310                 315                 320

Leu Asn Met Met Leu Asp Val Thr Phe Ile Ile Asn Gly Val Pro Tyr
                325                 330                 335

Thr Leu Asn Pro Thr Ala Tyr Ile Leu Leu Asp Phe Val Asp Gly Met
            340                 345                 350

Gln Val Cys Ser Thr Gly Phe Glu Gly Leu Glu Ile Gln Pro Pro Ala
        355                 360                 365

Gly Pro Leu Trp Ile Leu Gly Asp Val Phe Ile Arg Gln Phe Tyr Ala
    370                 375                 380

Val Phe Asp Arg Gly Asn Asn Arg Val Gly Leu Ala Pro Ala Val Pro
385                 390                 395                 400

<210>  4
<211>  400
<212>  PRT
<213>  Rabbit

<400>  4

Met Lys Thr Leu Xaa Pro Leu Leu Leu Leu Leu Xaa Leu Leu Asp Leu
                5                   10                  15

Gly Gln Ala Gln Gly Ile Leu Asp Arg Val Pro Leu Xaa Arg Arg Gln

20                  25                  30

Pro Ser Leu Arg Lys Lys Leu Arg Ala Gln Gly Gln Leu Ser Glu Phe
        35                  40                  45

Trp Lys Ala His Lys Val Asp Met Val Gln Tyr Thr Glu Thr Cys Thr
    50                  55                  60

Met Glu Gln Ser Ala Asn Glu Pro Leu Ile Asn Tyr Leu Asp Met Glu
65                  70                  75                  80

Tyr Phe Gly Thr Ile Ser Ile Gly Ser Pro Pro Gln Asn Phe Thr Val
                85                  90                  95

Ile Phe Asp Thr Val Ser Ser Asn Leu Trp Val Pro Ser Val Tyr Cys
            100                 105                 110

Thr Ser Pro Ala Cys Gln Met His Pro Gln Phe Arg Pro Ser Gln Ser
        115                 120                 125

Asn Thr Tyr Ser Glu Val Gly Thr Pro Phe Ser Ile Ala Tyr Gly Thr
    130                 135                 140

Gly Ser Leu Thr Gly Ile Ile Gly Ala Asp Gln Val Ser Val Gln Gly
145                 150                 155                 160

Leu Thr Val Val Gly Gln Gln Phe Gly Glu Ser Val Lys Glu Pro Gly
                165                 170                 175

Gln Thr Phe Val Asn Ala Glu Phe Asp Gly Ile Leu Gly Leu Gly Tyr
            180                 185                 190

Pro Ser Leu Ala Ala Gly Gly Val Thr Pro Val Phe Asp Asn Met Met
        195                 200                 205

Ala Gln Asn Leu Val Ser Leu Pro Met Phe Ser Val Tyr Met Ser Ser
    210                 215                 220

Asn Pro Glu Gly Gly Ser Gly Ser Glu Leu Thr Phe Gly Gly Tyr Asp
225                 230                 235                 240

Ser Ser His Phe Ser Gly Ser Leu Asn Trp Val Pro Val Thr Lys Gln
                245                 250                 255

Gly Tyr Trp Gln Ile Ala Leu Asp Glu Ile Gln Val Gly Gly Ser Pro
            260                 265                 270

Met Phe Cys Pro Glu Gly Cys Gln Ala Ile Val Asp Thr Gly Thr Ser
        275                 280                 285

Leu Ile Thr Gly Pro Ser Asp Lys Ile Ile Gln Leu Gln Ala Ala Ile
    290                 295                 300

Gly Ala Thr Pro Met Asp Xaa Gly Glu Tyr Ala Val Glu Cys Glu Asn
305                 310                 315                 320

Leu Asn Ile Met Pro Asp Val Thr Phe Val Ile Asn Gly Val Pro Tyr
                325                 330                 335

Thr Leu Ser Ala Thr Ala Tyr Thr Leu Pro Asp Phe Val Asp Gly Met
            340                 345                 350

Gln Phe Cys Gly Ser Gly Phe Gln Gly Leu Asp Ile Gln Pro Pro Ala
        355                 360                 365

Gly Pro Leu Trp Ile Leu Gly Asp Val Phe Ile Arg Gln Phe Tyr Ser
    370                 375                 380

Val Phe Asp Arg Gly Ser Asn Arg Val Gly Leu Ala Pro Ala Val Pro
385                 390                 395                 400